# EUROPEAN PATENT SPECIFICATION

(11) **EP 3 441 743 B1**
(45) Date of publication and mention of the grant of the patent: **13.09.2023**
(21) Application number: 18187469.4
(22) Date of filing: 06.08.2018
(51) Int. Cl.: G01N 15/14, G01N 15/10, G01N 21/64

(54) **SAMPLE ANALYZER AND SAMPLE ANALYZING METHOD**
PROBENANALYSATOR UND PROBENANALYSEVERFAHREN
ANALYSEUR D'ÉCHANTILLON ET PROCÉDÉ D'ANALYSE D'ÉCHANTILLON

(30) Priority: 10.08.2017 JP 2017155942
(43) Date of publication of application: 13.02.2019
(73) Proprietor: SYSMEX CORPORATION, Kobe-shi Hyogo 651-0073 (JP)
(72) Inventor: Konishi, Yusuke, Hyogo 651-0073 (JP); Yamada, Kazuhiro, Hyogo, 651-0073 (JP); Matsumoto, Shohei, Hyogo, 651-0073 (JP)
(74) Representative: Hoffmann Eitle

(56) References cited:
- EP-A1- 3 441 742
- EP-A1- 3 492 907
- WO-A1-2017/011549
- US-A1- 2003 235 919
- US-A1- 2006 068 371
- US-A1- 2010 254 590
- US-A1- 2011 282 870
- US-A1- 2016 025 621

## Description

### CROSS REFERENCE TO RELATED APPLICATIONS

This application claims priority from prior Japanese Patent Application No. 2017-155942, filed on August 10, 2017, entitled "SAMPLE ANALYZER AND SAMPLE ANALYZING METHOD".

### FIELD OF THE INVENTION

The present invention relates to a sample analyzer and a sample analyzing method that analyze a target substance contained in a measurement specimen.

### BACKGROUND

Japanese National Phase PCT Laid-Open Publication No. 2005-515408 discloses a cell processing method to be used when a flow cytometer or the like is applied to detection in a fluorescence in situ hybridization (FISH). In the FISH, a labeled probe is hybridized with a DNA sequence region in a cell, and fluorescence caused by the labeled probe is detected, thereby analyzing the cell.

In a case where analysis is performed for a measurement item, a labeling process is, for example, performed so as to generate fluorescences having different colors from a plurality of kinds of target substances, respectively, for the measurement item, in one cell. Various fluorescent dyes for labeling target substances are known. In this case, in order to cause the analyzer to automatically analyze a cell, the analyzer needs to obtain colors of fluorescences generated from a plurality of kinds of target substances, and then analyze the target substances. In a case where a plurality of measurement items are set for one cell, the analyzer needs to obtain colors of fluorescences generated by a plurality of kinds of target substances for the plurality of measurement items, and then analyze the target substances. In this case, the analyzer needs to obtain more kinds of colors of fluorescences.

In a case where the analyzer cannot obtain colors of fluorescences generated from a plurality of kinds of target substances, incorrect fluorescence may be associated with a target substance. In this case, accuracy for analysis may be reduced, or incorrect analysis result may be obtained. Therefore, an analyzer that can obtain, when a plurality of colors of fluorescences are generated, the colors of the fluorescences generated by target substances, and appropriately analyze the target substances, is required. In this regard, US 2011/282870 A1 discusses a system and method for selecting an optimal multi-marker reagent combination for the identification and/or quantification of molecules in or on cells with or without reference to fluorescence or other properties of at least one fluorescent dye or other instrument-measurable atom or molecule associated directly or indirectly with the reagent combination. This is achieved by specifying, using a computer, a plurality of markers to be detected by a plurality of reagents, generating, using a computer, a plurality of reagent combinations comprising the plurality of reagents to detect the plurality of markers, wherein the reagent combinations are ranked according to at least one user-defined or system-defined criterion, and selecting the optimal reagent combination or providing a rank-ordered list of combinations. Additionally, US 2016/025621 A1 discusses systems and methods for determining detection limits for various antibody-dye conjugates for flow cytometry. Herein, exemplary techniques involve a linear superpositioning approach of spill-over-induced enlargements of normally distributed measurement errors.

Also, WO 2017/011549 A1 discusses methods and systems for performing a flow cytometry experiment that can include evaluating one or more assignments of labels to a plurality of markers. Herein, the evaluation of assignments of labels can include evaluating condition numbers for at least one spillover matrix. Further, the method can further include selecting labels based at least in part on the evaluation of condition numbers. Methods and systems for performing a flow cytometry experiment can also include inputting data regarding markers, cell populations, labels, and cytometer configuration for a flow cytometry experiment, assigning labels to at least some of the markers, and running the flow cytometry experiment.

US 2006/068371 A1 discloses a sample analyzer for analyzing a measurement specimen containing a plurality of kinds of target substances each labeled with a fluorescent dye, the sample analyzer comprising: a measurement unit comprising: a flow cell allowing the measurement specimen to flow therethrough; a light source configured to apply light to the measurement specimen flowing through the flow cell to generate, from the fluorescent dye, a plurality of kinds of fluorescences having different wavelengths; an imaging unit configured to take an image of the fluorescence generated from a cell in the measurement specimen flowing through the flow cell, for each color of the fluorescence, and generate a fluorescence image; and a processing unit configured to analyze the target substances based on the fluorescence image and information on colors of fluorescences, wherein the analyzing the target substances comprises: receiving information on colors of fluorescences corresponding to the respective target substances; extracting a fluorescence region from the fluorescence image and obtaining a bright point pattern of the cell based on the fluorescence region and the information on colors of fluorescences. EP 3 441 742 A1 discloses another sample analyzer.

### SUMMARY OF THE INVENTION

A first aspect of the present invention is directed to a sample analyzer (10) according to claim 1 . The sample analyzer (10) according to this aspect includes: a measurement unit comprising: a flow cell allowing the measurement specimen to flow therethrough; a light source configured to apply light to the measurement specimen flowing through the flow cell to generate, from the fluorescent dye, a plurality of kinds of fluorescences having different wavelengths; an imaging unit configured to take an image of the fluorescence generated from a cell in the measurement specimen flowing through the flow cell, for each color of the fluorescence, and generate a fluorescence image; and a processing unit configured to analyze the target substances based on the fluorescence image and information on colors of fluorescences, which is set to be variable so as to correspond to the plurality of kinds of target substances, wherein the analyzing the target substances comprises: receiving information on colors of fluorescences corresponding to the respective target substances; obtaining a positive pattern and a negative pattern based on the information on colors of fluorescences, the positive pattern and the negative pattern being bright point patterns; extracting a fluorescence region from the fluorescence image and obtaining a bright point pattern of the cell based on the fluorescence region and the information on colors of fluorescences; and determining whether the cell is positive or negative by comparing the bright point pattern of the cell with the positive pattern and the negative pattern.

The "information on a color of fluorescence" represents information that allows discrimination among fluorescence generated from a target substance, and represents, for example, a color name of the fluorescence, a wavelength of the fluorescence, a kind of fluorescence labeling, a kind of a reagent based on fluorescence labeling, or a channel for analysis for each fluorescence. The "detection result" includes a taken image of fluorescence, intensity of fluorescence, and the like. In the sample analyzer according to this aspect, information on a color of fluorescence corresponding to a target substance is set so as to be variable. Therefore, information on a color of fluorescence is set so as to correspond to a target substance, whereby the processing unit can obtain the color of the fluorescence generated from the target substance. Therefore, the processing unit can appropriately analyze a plurality of kinds of target substances by using the detection results corresponding to the target substances.

The sample analyzer (10) according to this aspect includes a display unit (203), and the processing unit (201) may cause the display unit (203) to display an input screen (330) for receiving input of the information on the color of the fluorescence such that the information on the color of the fluorescence corresponds to the target substance. Thus, an operator is allowed to smoothly input the information on the color of the fluorescence through the input screen.

In this case, the input screen (330) may include a plurality of sets each including an item for the target substance and an item for inputting the information on the color of the fluorescence. Thus, also in a case where the number of target substances is plural, an operator is allowed to smoothly input information on a color of fluorescence for each of the plurality of kinds of target substances.

The target substance is related to a measurement item, the processing unit (201) may receive input of the measurement item, and the input screen (330) may include a set of an item for the target substance corresponding to the inputted measurement item, and an item for inputting the information on the color of the fluorescence. Thus, an operator is allowed to smoothly input, through a screen, information on a color of fluorescence for a target substance related to a measurement item.

The sample analyzer (10) according to this aspect includes an information obtaining unit (205) configured to obtain identification information (51, 61), and the processing unit (201) may receive input of the information on the color of the fluorescence, based on the identification information (51, 61) obtained by the information obtaining unit (205). When the identification information includes barcode information, the information obtaining unit is implemented by a barcode reader for reading the barcode. When the identification information is implemented by a RFID, the information obtaining unit is implemented by an antenna for reading the RFID from a RFID tag. When the identification information is implemented by a particular structure such as a cut or a hole, the information obtaining unit is implemented by a device for identifying the particular structure such as a cut or a hole. The identification information is provided in, for example, a reagent container, or a container that contains a plurality of reagent containers. Thus, an operator is allowed to smoothly input information on a color of fluorescence by using the information obtaining unit.

In this case, the sample analyzer (10) according to this aspect includes a storage unit (202) configured to store the information on the color of the fluorescence such that the information on the color of the fluorescence corresponds to the identification information (51, 61), and the processing unit (201) may receive input of the information on the color of the fluorescence which corresponds to the target substance by reading, from the storage unit (202), the information, on the color of the fluorescence, which corresponds to the identification information (51, 61) obtained by the information obtaining unit (205). Thus, an operator is allowed to smoothly input information on a color of fluorescence such that the information on the color of the fluorescence corresponds to the target substance by using the information obtaining unit.

In this case, the storage unit (202) may store the plurality of kinds of target substances related to the measurement item such that the plurality of kinds of target substances correspond to the identification information (61), and the processing unit (201) may receive input of the information on the colors of the fluorescences which correspond to the plurality of kinds of target substances related to the measurement item, by reading, from the storage unit (202), the measurement item which corresponds to the identification information (61) obtained by the information obtaining unit (205), and the information, on the colors of the fluorescences, corresponding to the identification information (61). Thus, an operator is allowed to smoothly input information on colors of fluorescences such that the information on the colors of the fluorescences correspond to a plurality of kinds of target substances related to the measurement item by using the information obtaining unit.

In the sample analyzer (10) according to this aspect, the information on the color of the fluorescence may represent at least one of a color name of the fluorescence, a wavelength of the fluorescence, a kind of fluorescence labeling, and a kind of a reagent based on the fluorescence labeling.

In the sample analyzer (10) according to this aspect, the measurement unit (100) further includes a flow cell (110) that allows the measurement specimen (21) containing the plurality of kinds of target substances to flow therethrough; a light source (121 to 125) that applies light to the measurement specimen (21) that flows through the flow cell (110); and an imaging unit (154) that takes an image of the fluorescence generated from a cell in the measurement specimen (21) that flows through the flow cell (110), for each color of the fluorescence, and generates a fluorescence image. Thus, since images of many cells in the measurement specimen are taken, the cells can be smoothly analyzed with accuracy.

In this case, the sample analyzer (10) according to this aspect includes a display unit (203), and the processing unit (201) may cause the display unit (203) to display the fluorescence image taken by the imaging unit (154). Thus, an operator is allowed to confirm location of the target substance, presence or absence of the target substance, an amplified state, and the like, with reference to the fluorescence image. Thus, the operator is allowed to determine whether a cell containing a target substance is positive or negative.

In this case, the processing unit (201) may combine a plurality of the fluorescence images taken by the imaging unit (154), based on the one cell, and cause the display unit (203) to display an obtained composite image. Thus, an operator is allowed to smoothly confirm locations of a plurality of kinds of target substances with reference to the composite image. Therefore, the operator is allowed to determine, for example, whether a cell containing a target substance is positive or negative.

In the sample analyzer (10) according to this aspect, the processing unit (201) determines whether each cell is positive or negative for a measurement item related to the target substance, based on the information on the color of the fluorescence and the fluorescence image.

The processing unit (201) extracts a fluorescence region based on the target substance, from the fluorescence image of the target substance related to the measurement item, and determines whether a result is positive or negative for the measurement item, based on the extracted fluorescence region. The fluorescence region includes not only a region, having a certain degree of area on the fluorescence image, obtained by fluorescence being applied to a wide region, but also a region, having such a small area as to be seen as a point on the fluorescence image, obtained by fluorescence being applied to a narrow region, that is, a bright point.

The processing unit (201) determines whether a result is positive or negative for the measurement item by comparing a location pattern in the extracted fluorescence region and a predetermined pattern with each other. Thus, the processing unit can determine, for example, whether genetic translocation is positive or negative for the measurement item related to genetic translocation.

The sample analyzer (10) according to this aspect includes a display unit (203), and the processing unit (201) may cause the display unit (203) to display a positive or a negative determination result for each cell. Thus, an operator is allowed to confirm the obtained determination result for each cell.

In this case, the processing unit (201) may cause the display unit (203) to display at least one of the number of positive cells, a percentage of positive cells, the number of negative cells, and a percentage of negative cells, based on the positive or the negative determination result for each cell. Thus, a doctor and the like are allowed to smoothly determine whether a specimen and a subject are positive or negative with reference to the displayed information.

In the sample analyzer (10) according to this aspect, the target substance is related to a measurement item, and the processing unit (201) may analyze each cell for a plurality of the measurement items, based on the information on the colors of the fluorescences and a detection result from the measurement unit (100, 500).

In this case, the sample analyzer (10) according to this aspect includes a display unit (203), and the processing unit (201) may cause the display unit (203) to display the measurement item for which the number of positive cells is greatest or a percentage of positive cells is highest. Thus, an operator is allowed to smoothly recognize a measurement item that is prominent due to the number of positive cells being great or a percentage of positive cells being high.

In the sample analyzer (10) according to this aspect, the measurement item related to the plurality of kinds of target substances is, for example, BCR-ABL, and the target substances related to the measurement item include, for example, BCR gene and ABL gene.

In the sample analyzer (10) according to this aspect, the measurement item related to the plurality of kinds of target substances is, for example, BCR-ABL and PML-RARα, and the target substances related to the measurement item include, for example, BCR gene, ABL gene, PML gene, and RARα gene.

A second aspect of the present invention is directed to a sample analyzing method. The sample analyzing method according to this aspect includes: setting information on colors of fluorescence which is a variable parameter such that the information on the colors of fluorescence corresponds to the plurality of kinds of target substances; flowing, through a flow cell, the measurement specimen; applying light to the measurement specimen flowing through the flow cell to generate, from the fluorescent dye, a plurality of kinds of fluorescences, having different wavelengths; taking an image of the fluorescence generated from a cell in the measurement specimen flowing through the flow cell, for each color of the fluorescence, and generating a fluorescence image; and analyzing the target substances based on the fluorescence image and the set information on the colors of fluorescences; wherein the analyzing the target substances comprises: receiving information on colors of fluorescences corresponding to the respective target substances; obtaining a positive pattern and a negative pattern based on the information on colors of fluorescences, the positive pattern and the negative pattern being bright point patterns; extracting a fluorescence region from the fluorescence image and obtaining a bright point pattern of the cell based on the fluorescence region and the information on colors of fluorescences; and determining whether the cell is positive or negative by comparing the bright point pattern of the cell with the positive pattern and the negative pattern.

The sample analyzing method according to this aspect enables the same effect as in the first mode to be obtained.

According to the present invention, a color of fluorescence generated from a target substance is obtained, and the target substance can be appropriately analyzed.

### BRIEF DESCRIPTION OF THE DRAWINGS

FIG. 1 schematically illustrates a structure of a sample analyzer according to Embodiment 1;
FIG. 2 schematically illustrates regions on a light receiving surface of an imaging unit according to Embodiment 1;
FIGS. 3A and FIG. 3B illustrate an outline of analysis performed by a processing unit according to Embodiment 1;
FIG. 4A is a flow chart showing a process for associating a target substance with information on a color of a fluorescence, according to Embodiment 1;
FIG. 4B illustrates a screen for receiving a measurement panel name according to Embodiment 1;
FIG. 4C illustrates a screen for receiving a measurement item according to Embodiment 1;
FIG. 5A illustrates an input screen for receiving information on a color of fluorescence according to Embodiment 1;
FIG. 5B illustrates a state where color names are displayed below a selection portion according to Embodiment 1;
FIG. 6A illustrates an input screen for receiving information on a color of fluorescence according to Embodiment 1;
FIG. 6B illustrates a screen displayed when color names in the selection portions in a list are used multiple times, according to Embodiment 1;
FIGS. 7A to FIG. 7C illustrate a basic color table and a basic pattern table according to Embodiment 1;
FIG. 8 is a flow chart showing measurement and analysis process according to Embodiment 1;
FIG. 9A and FIG. 9B illustrate extraction of a fluorescence region according to Embodiment 1;
FIG. 9C schematically illustrates fluorescence images and a composite image in a negative pattern according to Embodiment 1;
FIG. 9D schematically illustrates fluorescence images and a composite image in a positive pattern according to Embodiment 1;
FIG. 10 illustrates a screen for displaying a measurement result for each specimen according to Embodiment 1;
FIG. 11 illustrates a screen for displaying a measurement result for each measurement item according to Embodiment 1;
FIG. 12 illustrates a screen for displaying information on a measurement item for which the number of positive cells is greatest or a percentage of positive cells is highest, according to Embodiment 1;
FIG. 13 illustrates a screen for displaying an image according to Embodiment 1;
FIG. 14 illustrates a screen for displaying an image according to Embodiment 1;
FIG. 15 illustrates a screen for displaying an image according to Embodiment 1;
FIG. 16A illustrates a state where fluorescence wavelength bands are displayed below a selection portion according to Embodiment 2;
FIG. 16B illustrates a state where channels are displayed below a selection portion according to Embodiment 3;
FIG. 17A illustrates a screen for selecting a fluorescent dye name according to Embodiment 4;
FIG. 17B schematically illustrates a structure of a fluorescent dye database according to Embodiment 4;
FIG. 17C illustrates a screen for selecting a reagent name according to Embodiment 5;
FIG. 17D schematically illustrates a structure of a reagent database according to Embodiment 5;
FIG. 18 schematically illustrates a configuration of reagent containers, identification information, and an information obtaining unit according to Embodiment 6;
FIG. 19 schematically illustrates a configuration of a test kit container, identification information, and an information obtaining unit according to Embodiment 7;
FIG. 20 illustrates automatic setting of information on a color of fluorescence on an input screen according to Embodiment 8; and
FIG. 21 schematically illustrates a structure of a measurement unit not forming part of the present invention.

### DETAILED DESCRIPTION OF THE PREFERRED EMBODIMENTS

### <Embodiment 1>

In Embodiment 1, this disclosure is applied to an apparatus which measures and analyzes a measurement specimen prepared in preprocessing including a step of hybridizing a nucleic acid probe labeled with a fluorescent dye, with a gene in nucleic acid. The measurement specimen is a sample that contains a target substance. In Embodiment 1, the target substance is a nucleus of a cell to be detected, and one or a plurality of kinds of genes in the cell to be detected. A nucleus that is the target substance is detected by specifically staining the nucleus by a fluorescent dye for staining a nucleus. A gene in the target substance is detected by FISH. The target substance is not limited to a nucleus or a gene. The target substance may be, for example, a specific substance contained in a nucleus in a cell, a specific substance in a cell membrane, a cell membrane, a cell, a surface of a cell, or protein. Labeling of a target substance may be performed based on antigen-antibody reaction.

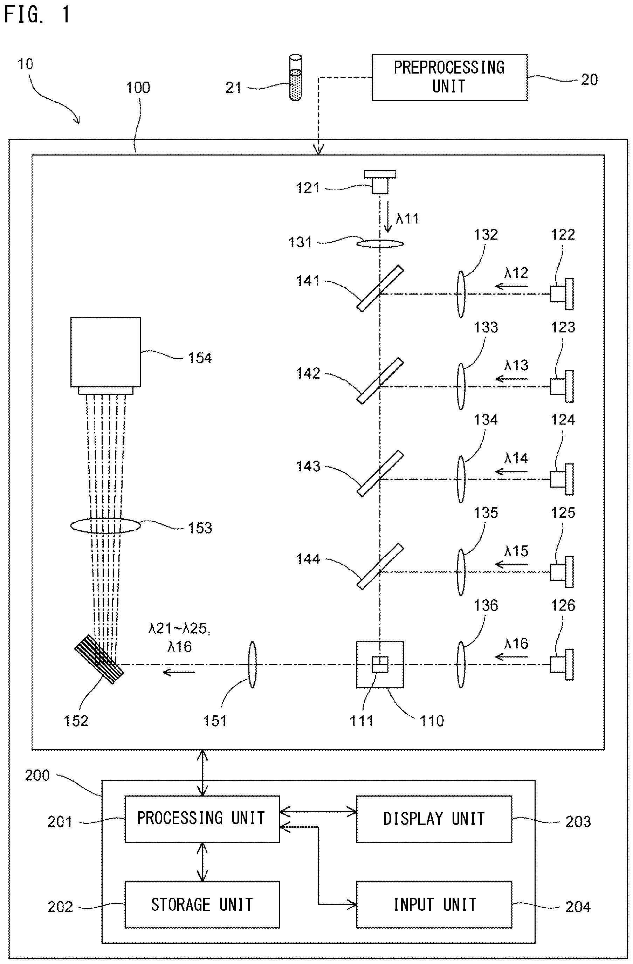

As shown in FIG. 1, a sample analyzer 10 measures and analyzes a measurement specimen 21 prepared in the preprocessing performed by a preprocessing unit 20.

The preprocessing unit 20 performs processing, such as centrifuging, of a whole blood specimen collected from a subject, and extracts white blood cells as cells to be detected. White blood cells may be extracted by hemolyzing other blood cells with the use of a hemolyzing agent, instead of centrifuging. The specimen may be plasma, cerebrospinal fluid, tissue fluid, or urine as well as whole blood collected from an organism. The cell to be detected is not limited to a white blood cell, and may be, for example, an epithelial cell.

The preprocessing unit 20 includes: a mixing container for mixing a reagent with a specimen having been subjected to the processing such as centrifuging; a dispensing unit for dispensing a specimen and a reagent into the mixing container; a heating unit for heating the mixing container; and the like. The preprocessing unit 20 prepares the measurement specimen 21 by performing the preprocessing including a step of labeling a gene in a cell to be detected with a fluorescent dye, and a step of staining a nucleus in the cell to be detected with a fluorescent dye for staining a nucleus. In the step of labeling the gene with the fluorescent dye, a nucleic acid probe labeled with the fluorescent dye, and the gene in a nucleic acid are hybridized with each other.

In Embodiment 1, for one cell, up to four genes are labeled with fluorescent dyes. The fluorescent dyes for labeling each gene and a nucleus generate fluorescences in different wavelength bands by applying excitation light.

Specifically, as described below, the sample analyzer 10 is structured so as to apply lights having wavelengths λ11, λ12, λ13, λ14, and λ15 as excitation light. The sample analyzer 10 can discriminate among fluorescences having wavelengths λ21, λ22, λ23, λ24, and λ25, and detect the fluorescences. Therefore, the fluorescent dye for labeling each of four genes and a nucleus is selected from among a fluorescent dye that generates fluorescence having the wavelength λ21 by application of excitation light having the wavelength λ11, a fluorescent dye that generates fluorescence having the wavelength λ22 by application of excitation light having the wavelength λ12, a fluorescent dye that generates fluorescence having the wavelength λ23 by application of excitation light having the wavelength λ13, a fluorescent dye that generates fluorescence having the wavelength λ24 by application of excitation light having the wavelength λ14, and a fluorescent dye that generates fluorescence having the wavelength λ25 by application of excitation light having the wavelength λ15.

For example, in a case where BCR gene, ABL gene, PML gene, and RARα gene are detected by one time measurement, the BCR gene is labeled with the fluorescent dye that generates fluorescence having the wavelength λ21 by application of excitation light having the wavelength λ11. The ABL gene is labeled with the fluorescent dye that generates fluorescence having the wavelength λ22 by application of excitation light having the wavelength λ12. The PML gene is labeled with the fluorescent dye that generates fluorescence having the wavelength λ23 by application of excitation light having the wavelength λ13. The RARα gene is labeled with the fluorescent dye that generates fluorescence having the wavelength λ24 by application of excitation light having the wavelength λ14. The nucleus is stained by a nucleus staining dye that generates fluorescence having the wavelength λ25 by application of excitation light having the wavelength λ15. Before measurement, an operator previously notifies the sample analyzer 10 in what wavelength bands the fluorescences, generated from the fluorescent dyes with which the genes and the nucleus have been labeled, are.

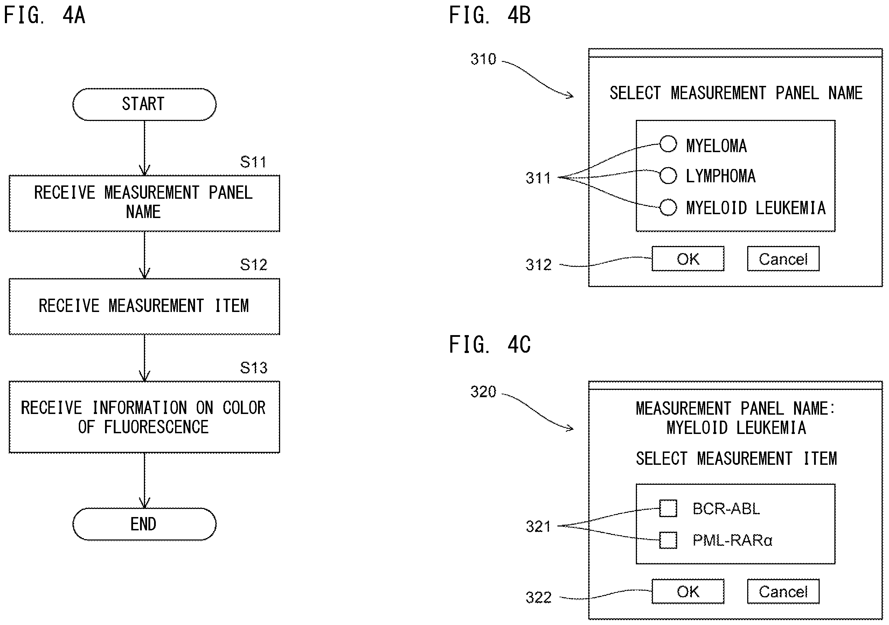

The sample analyzer 10 can appropriately perform analysis based on a gene since the sample analyzer 10 receives notification as to in what wavelength band the fluorescence, generated from the fluorescent dye with which the target substance is labeled, is. Association between target substances and colors of fluorescences generated by fluorescent dyes that label the target substances will be described below with reference to FIG. 4A and the subsequent drawings.

The sample analyzer 10 includes a measurement unit 100 and an analyzing unit 200. The measurement unit 100 includes a flow cell 110, light sources 121 to 126, condenser lenses 131 to 136, dichroic mirrors 141 to 144, a condenser lens 151, an optical unit 152, a condenser lens 153, and an imaging unit 154. The measurement unit 100 applies light to the measurement specimen 21 that contains a plurality of kinds of fluorescence-labeled target substances, and detects a plurality of kinds of fluorescences having different wavelengths. The measurement specimen 21 flows through a flow path 111 of the flow cell 110.

The light sources 121 to 126 apply light to the measurement specimen 21 that flows through the flow cell 110. The light sources 121 to 126 are implemented by semiconductor laser light sources. Lights emitted from the light sources 121 to 126 are laser lights having the wavelengths λ11 to λ16, respectively. The condenser lenses 131 to 136 condense lights emitted from the light sources 121 to 126, respectively. The dichroic mirror 141 allows light having the wavelength λ11 to pass therethrough, and reflects light having the wavelength λ12. The dichroic mirror 142 allows lights having the wavelengths λ11 and λ12 to pass therethrough and reflects light having the wavelength λ13. The dichroic mirror 143 allows lights having the wavelengths λ11 to λ13 to pass therethrough and reflects light having the wavelength λ14. The dichroic mirror 144 allows lights having the wavelengths λ11 to λ14 to pass therethrough and reflects light having the wavelength λ15. Thus, the lights having the wavelengths λ11 to λ16 are applied to the measurement specimen 21 that flows through the flow path 111 of the flow cell 110.

When the lights having the wavelengths λ11 to λ15 are applied to the measurement specimen 21 that flows through the flow cell 110, fluorescences are generated from fluorescent dyes with which the target substances are labeled. Specifically, in a case where four genes and the nucleus are labeled with the fluorescent dyes, each of the fluorescent dyes generates one of fluorescences having the wavelengths λ21 to λ25 as described above. In this case, when excitation lights having the wavelengths λ11 to λ15 are applied to the measurement specimen 21, fluorescences having the wavelengths λ21 to λ25 are generated from the measurement specimen 21. When light having the wavelength λ16 is applied to the measurement specimen 21 that flows through the flow cell 110, the light passes through the cell. The light, having the wavelength λ16, which has passed through the cell is used for generating a bright field image.

The condenser lens 151 condenses the fluorescences, having the wavelengths λ21 to λ25, generated from the measurement specimen 21, and the light, having the wavelength λ16, which has passed through the measurement specimen 21. The optical unit 152 has six dichroic mirrors combined with each other. The six dichroic mirrors of the optical unit 152 reflect the fluorescences having the wavelengths λ21 to λ25 and the light having the wavelength λ16 at slightly different angles, so as to separate the fluorescences and the light on the light receiving surface of the imaging unit 154. The condenser lens 153 condenses the fluorescences having the wavelengths λ21 to λ25 and the light having the wavelength λ16.

The imaging unit 154 includes a TDI (time delay integration) camera. The imaging unit 154 takes images of the fluorescences having the wavelengths λ21 to λ25 and the light having the wavelength λ16, and generates fluorescence images corresponding to the fluorescences having the wavelengths λ21 to λ25 and a bright field image corresponding to the light having the wavelength λ16. The imaging unit 154 transmits the generated taken images to the analyzing unit 200. The taken images generated by the imaging unit 154 are gray scale images.

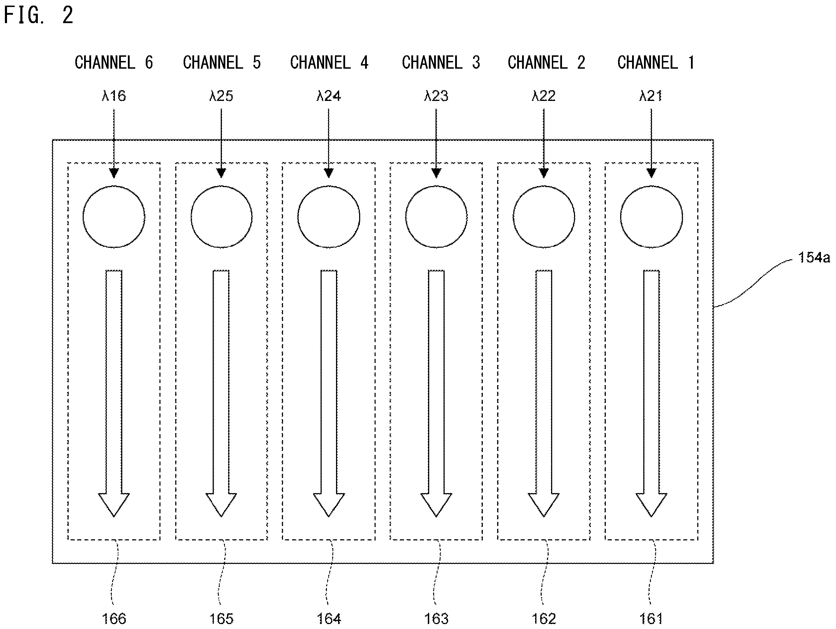

As shown in FIG. 2, the imaging unit 154 receives the lights having the wavelengths λ21 to λ25, and λ16 in regions 161 to 166, respectively, on a right receiving surface 154a. The light receiving surface 154a is a light receiving surface of an imaging element such as a CMOS image sensor disposed in the imaging unit 154. The positions of the lights applied to the light receiving surface 154a are changed in the regions 161 to 166, respectively, as indicated by outlined arrows in accordance with the cell being moved in the flow path 111 of the flow cell 110. Thus, the six lights are separated by the optical unit 152 on the light receiving surface 154a. Therefore, the imaging unit 154 can generate taken images corresponding to the respective lights. Channels 1 to 6 form a processing system in the sample analyzer 10 based on lights incident on regions 161 to 166, respectively. Images of lights having the wavelengths λ21 to λ25 and λ16 are taken in the channels 1 to 6, respectively, and analyzed.

The wavelengths λ21 to λ25, and λ16 of lights which are incident on the regions 161 to 166 are wavelengths different from each other. In Embodiment 1, for example, the fluorescence, having the wavelength λ21, which is incident on the region 161 has blue color. The fluorescence, having the wavelength λ22, which is incident on the region 162 has orange color. The fluorescence, having the wavelength λ23, which is incident on the region 163 has green color. The fluorescence, having the wavelength λ24, which is incident on the region 164 has red color. The fluorescence, having the wavelength λ25, which is incident on the region 165 is purple fluorescence. That is, in Embodiment 1, fluorescences, having the wavelengths λ21 to λ25, received by the imaging unit 154 in the respective regions, are fluorescences in blue, orange, green, red, and purple wavelength bands. The fluorescences having the wavelengths λ21 to λ25 are not limited to fluorescences having the above-described colors, and may be set to have other colors as long as the wavelengths λ21 to λ25 are in wavelength bands different from each other.

Returning to FIG. 1, the analyzing unit 200 includes a processing unit 201, a storage unit 202, a display unit 203, and an input unit 204.

The processing unit 201 is implemented by a CPU. The processing unit 201 may be implemented by a CPU and a microcomputer. The processing unit 201 performs various processes based on a program stored in the storage unit 202. The processing unit 201 is connected to the measurement unit 100, the storage unit 202, the display unit 203, and the input unit 204, and receives a signal from each unit and controls each unit. The storage unit 202 is implemented by a RAM, a ROM, a hard disk, and the like. The display unit 203 is implemented by a display. The input unit 204 is implemented by a mouse and a keyboard. The display unit 203 and the input unit 204 may be integrated with each other as, for example, a touch panel type display.

The processing unit 201 receives input of information on a color of fluorescence generated from a target substance, through the input unit 204, from an operator. The information on a color of fluorescence is information that can allow discrimination among fluorescences generated from the target substances. In Embodiment 1, the processing unit 201 receives a color name associated with a target substance, as information on a color of fluorescence. The color names represent colors of fluorescences incident on the regions 161 to 165 in the imaging unit 154, and represent blue, orange, green, red, and purple.

The processing unit 201 controls the measurement unit 100 so as to measure the measurement specimen 21 and obtain fluorescence images and a bright field image taken by the imaging unit 154. The processing unit 201 causes the storage unit 202 to store the obtained fluorescence images and bright field image. The processing unit 201 analyzes the target substances based on the inputted information on the colors of fluorescences and the fluorescence images obtained by the imaging unit 154.

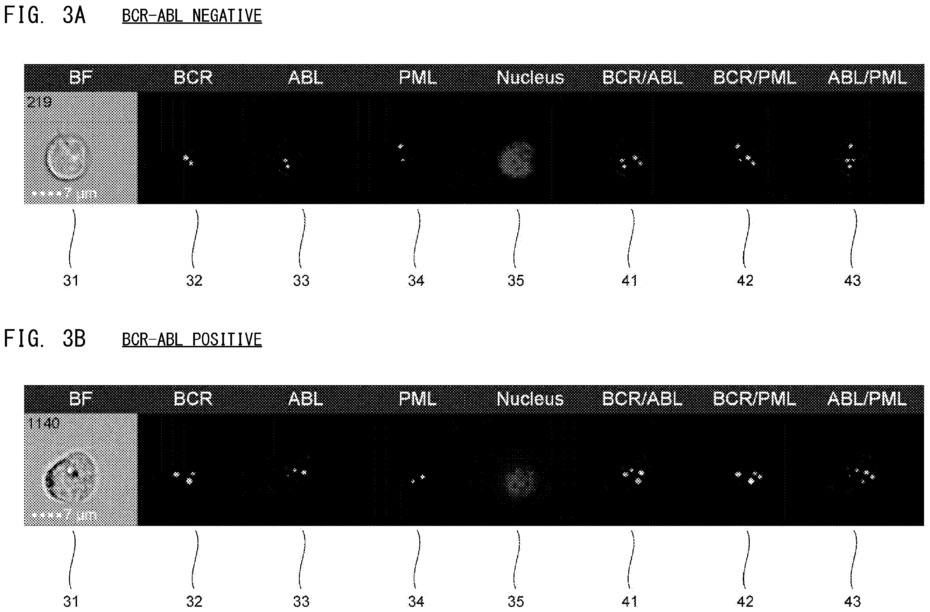

An outline of analysis performed by the processing unit 201 will be described with reference to FIGS. 3A and 3B.

A bright field image 31 is a bright field image taken by the imaging unit 154. Fluorescence images 32 to 35 are fluorescence images taken in different regions on the light receiving surface 154a of the imaging unit 154. A composite image 41 is obtained, by the processing unit 201, by combining the fluorescence images 32 and 33 with each other. A composite image 42 is obtained, by the processing unit 201, by combining the fluorescence images 32 and 34 with each other. A composite image 43 is obtained, by the processing unit 201, by combining the fluorescence images 33 and 34 with each other.

The bright field image 31 and the fluorescence images 32 to 35 shown in FIG. 3A and FIG. 3B are images taken based on the measurement specimen 21 in which BCR gene, ABL gene, PML gene, and the nucleus are fluorescence-labeled. The processing unit 201 detects, as an abnormal cell, a cell in which the BCR gene or the ABL gene has been translocated to form BCR-ABL fusion gene, based on the taken image. In this case, the processing unit 201 detects the abnormal cell by using the fluorescence image 32 corresponding to the BCR gene and the fluorescence image 33 corresponding to the ABL gene.

In FIG. 3A, the number of bright points of the BCR gene in the fluorescence image 32 is two, and the number of bright points of the ABL gene in the fluorescence image 33 is two. As indicated in the fluorescence images 32 and 33 and the composite image 41, the bright points of the BCR gene and the bright points of the ABL gene do not overlap each other. In this case, the processing unit 201 determines that no translocation has occurred, and determines that the cell to be detected is not an abnormal cell. Meanwhile, in FIG. 3B, the number of bright points of the BCR gene in the fluorescence image 32 is three, and the number of bright points of the ABL gene in the fluorescence image 33 is two. As indicated in the fluorescence images 32 and 33, and the composite image 41, a part of the bright points of the BCR gene and a part of the bright points of the ABL gene overlap each other. In this case, the processing unit 201 determines that translocation has occurred, and determines that the cell to be detected is an abnormal cell.

In such a determination, the obtained fluorescence images 32 to 34 need to be associated with the BCR gene, the ABL gene, and the PML gene, respectively. If the obtained fluorescence images 32 to 34 are associated with incorrect genes, the processing unit 201 cannot appropriately determine whether or not the BCR-ABL fusion gene has occurred. For example, if the BCR gene is associated with the fluorescence image 33, and the ABL gene is associated with the fluorescence image 34, the processing unit 201 detects the BCR-ABL fusion gene by using fluorescence images corresponding to the ABL gene and the PML gene. The composite image referred to in this case may be not the correct composite image 41 but the incorrect composite image 43. Thus, unless the processing unit 201 obtains association between the target substance and the fluorescence image in advance, the processing unit 201 cannot perform appropriate determination.

Meanwhile, in Embodiment 1, the processing unit 201 receives information on colors of fluorescences generated from the target substances, that is, color names associated with the target substances, through the input unit 204, from an operator. Therefore, the processing unit 201 obtains the colors of the fluorescences generated from the target substances, and can appropriately associate the gene of the target substance with the obtained fluorescence image. Thus, the processing unit 201 can appropriately analyze the target substance based on the inputted information on the color of the fluorescence, and the fluorescence image obtained by the imaging unit 154.

Next, a process of associating a target substance with information on a color of fluorescence will be described with reference to a flow chart shown in FIG. 4A. The process shown in FIG. 4A is typically performed before measurement of the measurement specimen 21. However, the process shown in FIG. 4A may be performed after the measurement of the measurement specimen 21.

In step S11, the processing unit 201 receives a measurement panel name from an operator through the input unit 204. Specifically, the processing unit 201 displays a screen 310 shown in FIG. 4B, on the display unit 203, and receives a measurement panel name through the screen 310.

As shown in FIG. 4B, the screen 310 includes selection portions 311 and an OK button 312. The selection portion 311 is provided for each measurement panel name. In Embodiment 1, the measurement panel names include myeloma, lymphoma, and myeloid leukemia. The selection portion 311 is implemented by a radio button. An operator is allowed to select only one of a plurality of the selection portions 311. The operator selects the selection portion 311 corresponding to the target measurement panel name, and then operates the OK button 312. Thus, the processing unit 201 causes the storage unit 202 to store the selected measurement panel name.

In Embodiment 1, only three measurement panel names can be selected. However, a measurement panel name other than the measurement panel names shown in FIG. 4B may be selected. A new measurement panel name may be added by an operator performing a predetermined operation on the input unit 204, and the selection portion 311 may be provided so as to correspond to the added measurement panel name.

In step S12, the processing unit 201 receives a measurement item through the input unit 204 from the operator. Specifically, the processing unit 201 operates so as to display a screen 320 shown in FIG. 4C, on the display unit 203, and receive a measurement item through the screen 320.

As shown in FIG. 4C, the screen 320 includes selection portions 321 and an OK button 322. The selection portion 321 is provided for each measurement item. The measurement items displayed on the screen 320 are displayed so as to correspond to the measurement panel name selected on the screen 310 shown in FIG. 4B. In the example shown in FIG. 4C, myeloid leukemia is selected as the measurement panel name. Therefore, "BCR-ABL" related to translocation of the BCR gene and the ABL gene, and "PML-RARα" related to translocation of the PML gene and the RARα gene, are displayed as the measurement items.

When myeloma is selected as the measurement panel name, "IGH-CCND1" related to translocation of IGH gene and CCND1 gene, "IGH-FGFR3" related to translocation of IGH gene and FGFR3 gene, and "IGH-cMAF" related to translocation of IGH gene and cMAF gene, are displayed as the measurement items. When lymphoma is selected as the measurement panel name, "IGH-CCND1" related to translocation of IGH gene and CCND1 gene, "IGH-MYC" related to translocation of IGH gene and MYC gene, and "IGH-BCL2" related to translocation of IGH gene and BCL2 gene are displayed as the measurement items.

The selection portions 321 include check boxes. The operator is allowed to select one or more selection portions 321 from a plurality of the selection portions 321. The operator selects the selection portion 321 corresponding to the target measurement item, and then operates the OK button 322. Thus, the processing unit 201 causes the storage unit 202 to store the selected measurement item.

In Embodiment 1, the measurement panel names are associated with the measurement items described above. However, other measurement items may be further associated according to, for example, diseases indicated by the measurement panel names. When an operator performs a predetermined operation through the input unit 204, a new measurement item may be added, and the selection portion 321 may be provided so as to correspond to the added measurement item.

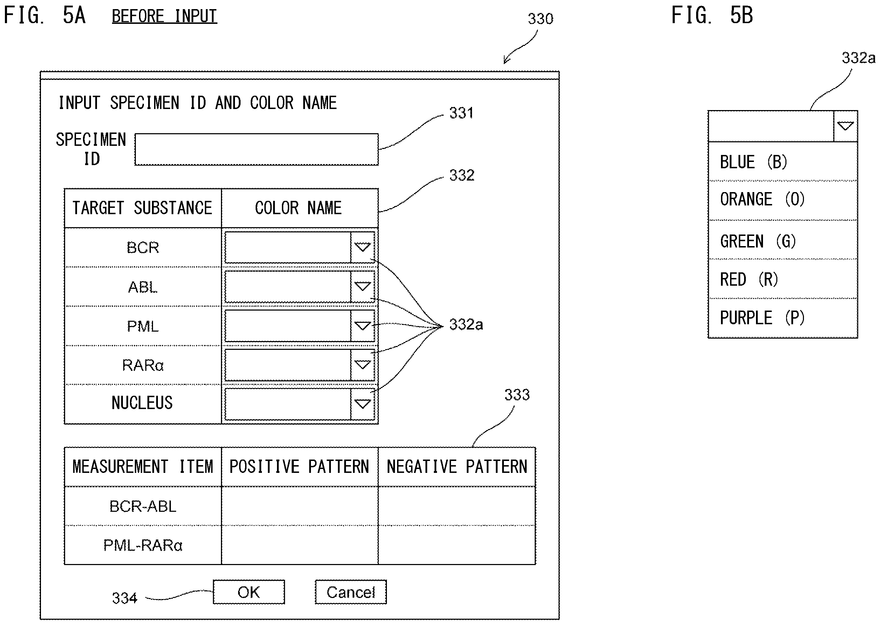

In step S13, the processing unit 201 receives, through the input unit 204, information on colors of fluorescences from the operator. Specifically, the processing unit 201 causes the display unit 203 to display an input screen 330 shown in FIG. 5A, and receives, through the input screen 330, information on colors of fluorescences.

As shown in FIG. 5A, the input screen 330 includes an input region 331, a list 332, a list 333, and an OK button 334.

The operator selects the input region 331, and then inputs a specimen ID for identifying a specimen to be measured, through the input unit 204. Thus, the inputted specimen ID is displayed in the input region 331.

In the list 332, association between target substances and information on colors of fluorescences is displayed. The target substance indicated in the list 332 is displayed so as to correspond to the measurement item selected in the screen 320 shown in FIG. 4C. In the example shown in FIG. 5A, since "BCR-ABL" and "PML-RARα" are selected as measurement items, "BCR" representing BCR gene, "ABL" representing ABL gene, "PML" representing PML gene, "RARα" representing RARα gene, and "nucleus" are displayed as the target substances.

In a case where "IGH-CCND1", "IGH-FGFR3", and "IGH-cMAF" are selected as the measurement items, "CCND1" representing CCND1 gene, "FGFR3" representing FGFR3 gene, "cMAF" representing cMAF gene, "IGH" representing IGH gene, and "nucleus" are displayed as the target substances. In a case where "IGH-CCND1", "IGH-MYC", and "IGH-BCL2" are selected as the measurement items, "CCND1" representing CCND1 gene, "MYC" representing MYC gene, "BCL2" representing BCL2 gene, "IGH" representing IGH gene, and "nucleus" are displayed as the target substances.

The list 332 includes selection portions 332a. The selection portion 332a is provided for each target substance to be displayed, and is implemented by a pull-down menu. In Embodiment 1, when the selection portion 332a is operated, a plurality of color names are displayed as information representing colors of fluorescences below the selection portion 332a, as shown in FIG. 5B. The color names displayed below the selection portion 332a correspond to the colors of fluorescences received by the regions 161 to 165 of the imaging unit 154 described above. The operator operates one of the plurality of color names displayed below the selection portion 332a, to select the color name associated with the target substance.

The input screen 330 is structured so as to receive information on a color of fluorescence as described above. Thus, the operator is allowed to smoothly input the information on a color of fluorescence through the input screen 330. The list 332 includes a plurality of sets each including an item of the target substance and an item for inputting information on a color of fluorescence. Thus, also when the number of the target substances is plural, the operator is allowed to smoothly input information on a color of fluorescence for each of the plurality of kinds of target substances. The input screen 330 includes a set of the item for the target substance corresponding to the measurement item having been inputted through the screen 320, and an item for inputting information on a color of fluorescence. Thus, the operator is allowed to smoothly input information on a color of fluorescence, for the target substance corresponding to the measurement item, through the input screen 330.

In the list 333, a positive pattern and a negative pattern for the measurement item are displayed. The measurement item displayed in the list 333 is the measurement item selected in the screen 320 shown in FIG. 4C. In the example shown in FIG. 5A, "BCR-ABL" and "PML-RARα" are selected as the measurement items on the screen 320, whereby "BCR-ABL" and "PML-RARα" are displayed.

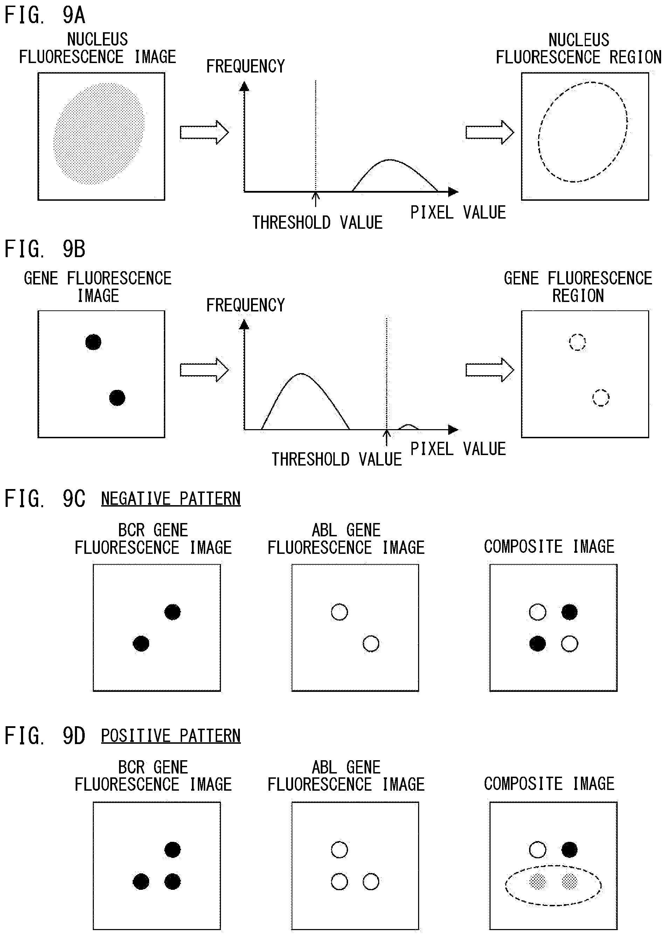

For the positive pattern, a bright point pattern for determining that a cell to be detected is positive for the measurement item is displayed. For the negative pattern, a bright point pattern for determining that a cell to be detected is negative for the measurement item is displayed. The positive pattern and the negative pattern for the measurement item are automatically displayed when, in the list 332, the selection portion 332a corresponding to the measurement item is operated, and a color name is thus selected. The bright point pattern will be described below with reference to FIG. 9C and FIG. 9D.

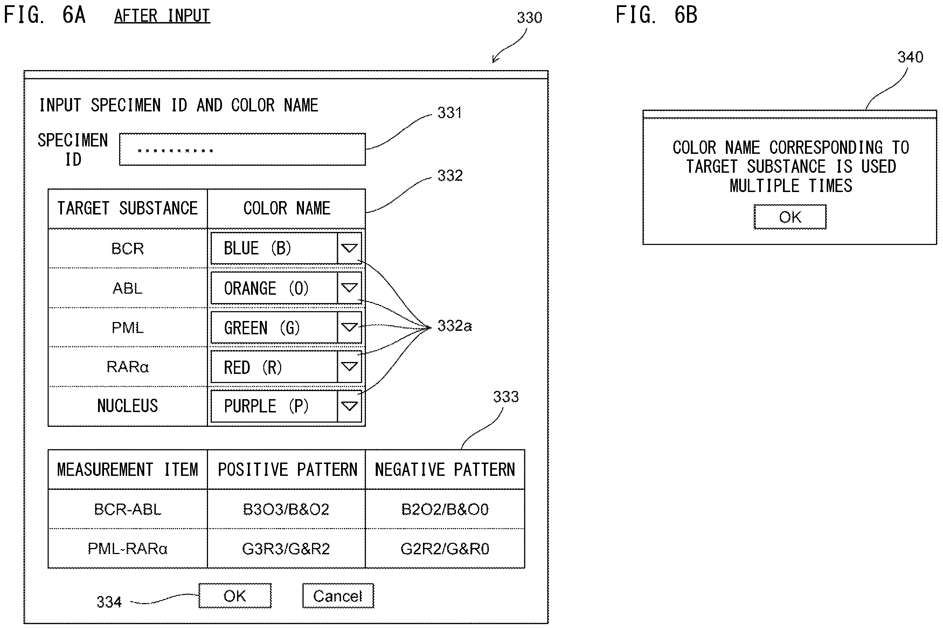

FIG. 6A illustrates a state where, in the input screen 330 shown in FIG. 5A, the specimen ID is inputted in the input region 331 and color names are selected for all the target substances.

In the example shown in FIG. 6A, blue, orange, green, red, and purple are selected as the color names for BCR gene, ABL gene, PML gene, RARα gene, and nucleus, respectively, which are the target substances. Thus, "B3O3/B&O2" and "B2O2/B&O0" are displayed for the positive pattern and the negative pattern, respectively, for the measurement item "BCR-ABL" in the list 333. In the item of the positive pattern and the item of the negative pattern for the measurement item "PML-RARα" in the list 333, "G3R3/G&R2" and "G2R2/G&R0", respectively, are displayed.

The positive pattern and the negative pattern are displayed in the list 333, by the processing unit 201, based on a table in which basic color names are registered, and a table in which the positive patterns and the negative patterns for the basis color names are registered.

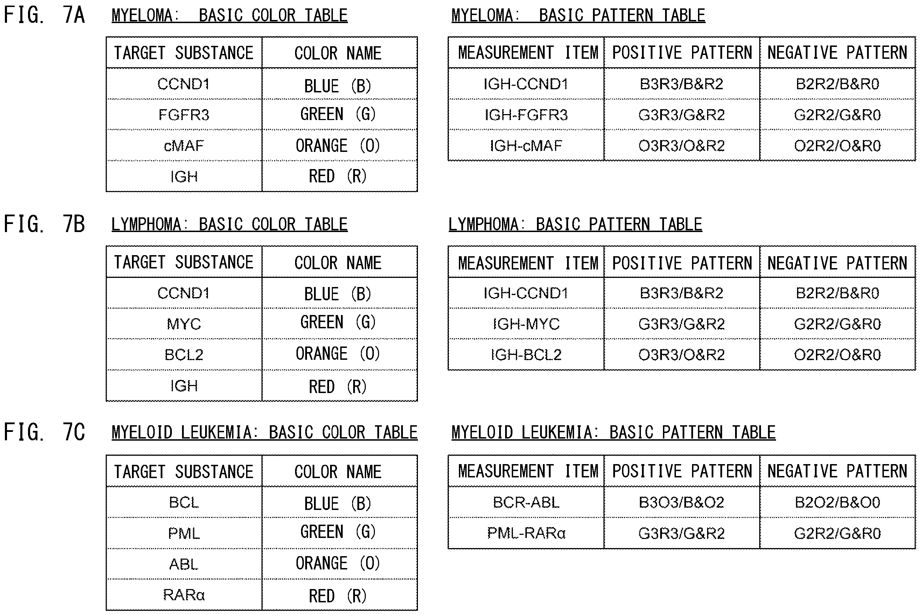

FIGS. 7A to 7C show tables used when the panel names are myeloma, lymphoma, and myeloid leukemia, respectively. In FIGS. 7A to 7C, the tables on the left side are basic color tables in which basis color names are registered, and the tables on the right side are basic pattern tables in which the positive patterns and the negative patterns corresponding to the basic color tables are registered. These tables are previously stored in the storage unit 202.

For example, in a case where the panel name is myeloid leukemia, blue, orange, green, and red are registered as the basis color names, for BCR gene, ABL gene, PML gene, and RARα gene, respectively, which are the target substances, as indicated in the basic color table shown in FIG. 7C. As the positive pattern and the negative pattern in the case of the target substances and the color names being associated with each other as registered in the basic color table, the positive patterns and the negative patterns for BCR-ABL and PML-RARα are registered as indicated in the basic pattern table shown in FIG. 7C.

In the basic pattern table, "B", "G", "R", "O", and "P" represent bright points on the fluorescence image based on blue, orange, green, red, and purple colors, respectively. The indication used for the pattern is defined as follows. For example, "B303" indicates that three bright points are on a blue fluorescence image, and three bright points are on an orange fluorescence image. "B&O2" indicates that the number of sets each including a bright point on a blue fluorescence image and a bright point on an orange fluorescence image such that the bright points overlap each other, is two. "/" indicates that two states are both satisfied.

Therefore, "B3O3/B&O2" indicates a pattern in which three bright points are on a blue fluorescence image, three bright points are on an orange fluorescence image, and the number of sets each including a bright point on a blue fluorescence image and a bright point on an orange fluorescence image such that the bright points overlap each other, is two. "B2O2/B&O0" indicates a pattern in which two bright points are on a blue fluorescence image, two bright points are on an orange fluorescence image, and the number of sets each including a bright point on a blue fluorescence image and a bright point on an orange fluorescence image such that the bright points overlap each other, is zero.

When the color name is selected for each target substance through the selection portion 332a of the input screen 330, the processing unit 201 generates the positive pattern and the negative pattern for each measurement item by using the tables shown in FIGS. 7A to 7C.

Specifically, the processing unit 201 reads the color name of the target substance related to the measurement item displayed in the list 333 of the input screen 330, from the basic color table for the selected measurement panel name. The processing unit 201 reads the positive pattern and the negative pattern related to the measurement item displayed in the list 333 of the input screen 330, from the basic pattern table for the selected measurement panel name. The processing unit 201 generates the positive pattern and the negative pattern to be displayed in the list 333, based on the color name read from the basic color table, the pattern read from the basic pattern table, and the color name selected in the selection portion 332a of the input screen 330. The generated positive pattern and negative pattern are displayed in the list 333 by the processing unit 201.

For example, in the example shown in FIG. 6A, the color names selected by the selection portion 332a are identical to those in the basic color table shown in FIG. 7C, and, therefore, the same contents as in the basic pattern table shown in FIG. 7C are displayed in the list 333.

Meanwhile, in a case where orange, green, red, and purple are selected as the color names corresponding to BCR gene, ABL gene, PML gene, and RARα gene, respectively, unlike the selected contents in the selection portion 332a as shown in FIG. 6A, the contents displayed in the list 333 are also different from those shown in FIG. 6A. That is, "O3G3/O&G2" and "O2G2/O&G0" are displayed as the positive pattern and the negative pattern, respectively, corresponding to the measurement item BCR-ABL in the list 333. "R3P3/R&P2" and "R2P2/R&P0" are displayed as the positive pattern and the negative pattern, respectively, corresponding to the measurement item PML-RARα in the list 333. Thus, the positive pattern and the negative pattern are displayed in the list 333 based on the selected contents in the selection portion 332a and each table.

The operator inputs the specimen ID in the input region 331, operates the selection portion 332a for each target substance to select the color name, as shown in FIG. 6A, and then operates the OK button 334. Thus, the processing unit 201 causes the storage unit 202 to store the specimen ID displayed in the input region 331, the color name, for each target substance, displayed in the list 332, and the positive pattern and the negative pattern, for each measurement item, displayed in the list 333.

In a case where, when the OK button 334 is operated, the color name in the selection portions 332a in the list 332 is used multiple times, the processing unit 201 causes the display unit 203 to display a screen 340 shown in FIG. 6B. As shown in FIG. 6B, the screen 340 indicates that "color name corresponding to target substance is used multiple times". Thus, the operator is notified of erroneous setting of the color name, whereby the operator is allowed to have an opportunity for setting a correct color name. Therefore, analysis using an incorrect color name can be inhibited.

In the basic pattern tables shown in FIG. 7A to FIG. 7C, one positive pattern is registered for each measurement item. However, two or more positive patterns may be registered for each measurement item.

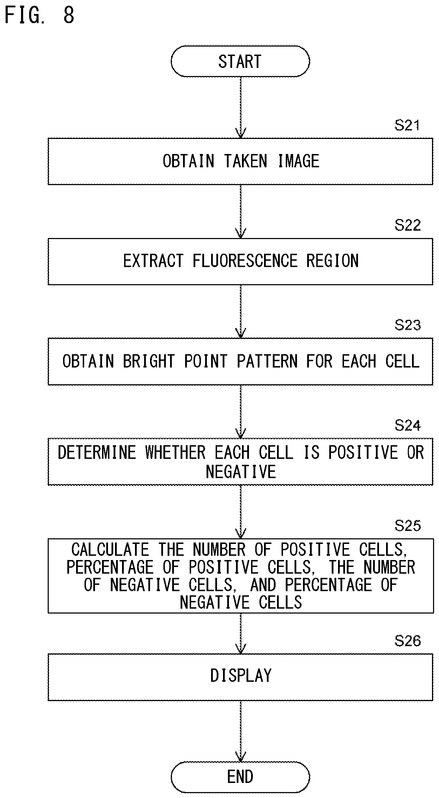

Next, measurement and analysis process will be described with reference to a flow chart shown in FIG. 8. The process shown in FIG. 8 is started by an operator inputting start instruction through the input unit 204.

In step S21, the processing unit 201 obtains a taken image. Specifically, the processing unit 201 controls the measurement unit 100 such that the measurement specimen 21 flows through the flow cell 110 and light is applied from the light sources 121 to 126 to the measurement specimen 21 flowing through the flow cell 110. Thus, fluorescence, having the wavelength λ21, which is obtained by excitation by light having the wavelength λ11, fluorescence, having the wavelength λ22, which is obtained by excitation by light having the wavelength λ12, fluorescence, having the wavelength λ23, which is obtained by excitation by light having the wavelength λ13, fluorescence, having the wavelength λ24, which is obtained by excitation by light having the wavelength λ14, and fluorescence, having the wavelength λ25, which is obtained by excitation by light having the wavelength λ15 are incident on the regions 161 to 165, respectively, of the imaging unit 154. The light having the wavelength λ16 passes through the measurement specimen 21 and is incident on the region 166 of the imaging unit 154.

The imaging unit 154 takes an image of the fluorescence incident on each of the regions 161 to 165 to generate a fluorescence image, and takes an image of transmitted light which is incident on the region 166 to generate a bright field image. The processing unit 201 obtains the fluorescence images and the bright field image generated by the imaging unit 154, and causes the storage unit 202 to store the obtained images.

The processing unit 201 causes the storage unit 202 to store the color name, for each target substance, inputted through the input screen 330 shown in FIG. 6A. Therefore, the processing unit 201 can obtain correspondence indicating to what target substance each of the fluorescence images based on fluorescences incident on the regions 161 to 165 corresponds. The processing unit 201 can use, in analysis of a target substance, an appropriate fluorescence image corresponding to the target substance, whereby the target substance can be appropriately analyzed.

In step S22, the processing unit 201 uses the fluorescence image obtained in step S21 to extract a fluorescence region based on a gene and a nucleus on the fluorescence image.

As indicated on the left end in FIG. 9A, in a case where a fluorescence image based on a nucleus is obtained, the processing unit 201 generates a graph in which pixel values are plotted against the frequency based on the pixel value of each pixel on the fluorescence image, as indicated at the center in FIG. 9B. The frequency represented by the vertical axis indicates the number of pixels. The processing unit 201 sets a threshold value for the pixel value in the graph. The processing unit 201 extracts, as a fluorescence region of the nucleus, a range in which pixels having pixel values greater than the threshold value are distributed, as indicated by dashed lines at the right end in FIG. 9A. Thus, the fluorescence region of the nucleus is a region having a certain degree of area on the fluorescence image. In a case where two nucleuses overlap each other in the fluorescence image of the nucleus, the corresponding cell is excluded and is not used for abnormal cell determination.

As indicated on the left end in FIG. 9B, in a case where a fluorescence image based on a gene is obtained, the processing unit 201 generates a graph in which pixel values are plotted against the frequency based on the pixel value of each pixel on the fluorescence image, as indicated at the center in FIG. 9B. The processing unit 201 sets a threshold value for a pixel value as a boundary between a bright point and a background, in the graph, according to, for example, the Otsu method. The processing unit 201 extracts, as a fluorescence region of the gene, a range in which pixels having pixel values greater than the threshold value are distributed, as indicated by dashed lines at the right end in FIG. 9B. Thus, the fluorescence region of the gene is a region having such a small area as to be seen as a point on the fluorescence image. Hereinafter, the fluorescence region of a gene is referred to as "bright point". When the bright point is extremely small, when the bright point is extremely large, and when the bright point is not included in the fluorescence region of the nucleus indicated on the right end in FIG. 9A, the corresponding cell is excluded and is not used for abnormal cell determination.

The processing unit 201 may extract the fluorescence region of a nucleus and a bright point of a gene according to calculation in the above-described procedure without generating the graph as indicated at the center in FIG. 9A and 9B.

The "pixel value", used in the above description, represents a digital value allocated to each pixel of the image. In Embodiment 1, the pixel value corresponds to an intensity of fluorescence generated by a fluorescent dye that labels a target substance or an intensity of bright field light that has passed through a cell. In the fluorescence images based on a nucleus and a gene, the pixel values represent values obtained by brightnesses of fluorescences generated from the fluorescent dyes that stain the nucleus and the gene being converted to digital signals, respectively. In the bright field image based on a cell, the pixel value represents a value obtained by brightness of light that has passed through the cell when light is applied being converted to a digital signal.

In step S23, the processing unit 201 obtains, for each cell, a bright point pattern from a bright point of a gene related to the selected measurement item. For example, in a case where genes related to the measurement item are "first gene" and "second gene", the processing unit 201 obtains, as the bright point pattern, the number of bright points of the first gene, the number of bright points of the second gene, and number of sets each including the bright point of the first gene and the bright point of the second gene such the bright points overlap each other, based on the bright points of the first gene and the bright points of the second gene, which are obtained in step S22.

FIGS. 9C and 9D schematically show images obtained when BCR-ABL is selected as the measurement item. The fluorescence images indicated at the left end in FIGS. 9C and 9D represent fluorescence images in a state where the fluorescence images of the BCR gene are colored with a color based on the color name. The fluorescence images indicated at the center in FIGS. 9C and 9D represent fluorescence images in a state where the fluorescence images of the ABL gene are colored with a color based on the color name.

In a case where the fluorescence images indicated at the left end and the center in FIG. 9C are obtained as the fluorescence image of BCR gene and the fluorescence image of ABL gene, the processing unit 201 obtains two as the number of the bright points of the BCR gene, two as the number of the bright points of the ABL gene, and zero as the number of sets each including the bright point of the BCR gene and the bright point of the ABL gene such that the bright points overlap each other. In this case, when the fluorescence image of the BCR gene and the fluorescence image of the ABL gene overlap each other, the composite image is obtained as indicated on the right end in FIG. 9C. As indicated in the composite image, the bright point of the BCR gene and the bright point of the ABL gene are distributed so as not to overlap each other in this case.

Meanwhile, in a case where the fluorescence images indicated at the left end and the center in FIG. 9D are obtained as the fluorescence image of the BCR gene and the fluorescence image of the ABL gene, the processing unit 201 obtains three as the number of the bright points of the BCR gene, three as the number of the bright points of the ABL gene, and two as the number of sets each including the bright point of the BCR gene and the bright point of the ABL gene such that the bright points overlap each other. In this case, when the fluorescence image of the BCR gene and the fluorescence image of the ABL gene overlap each other, the composite image is obtained as indicated on the right end in FIG. 9D. As indicated in the composite image, the bright points of the BCR gene and the bright points of the ABL gene are distributed such that a part the bright points of the BCR gene and a part of the bright points of the ABL gene overlap each other in a region indicated by dashed lines in this case. For example, when the bright point of the BCR gene is red, and the bright point of the ABL gene is green, a yellow bright point is generated by the red bright point and the green bright point being combined with each other in a portion in which the bright points overlap each other in the composite image.

Also in a case where another measurement item is selected, the processing unit 201 similarly obtains, for each cell, a bright point pattern from the bright points of the genes related to the selected measurement item.

In step S24, the processing unit 201 determines, for each cell, whether the cell is positive or negative for the selected measurement item. Specifically, the processing unit 201 compares the bright point pattern, corresponding to the measurement item, obtained in step S23, with contents of the positive pattern and the negative pattern of the measurement item displayed in the list 333 in FIG. 6A, and determines whether each cell is positive or negative. As shown in FIG. 9D, when the bright point pattern coincides with the positive pattern, the processing unit 201 determines that the target cell is positive for the measurement item, that is, the target cell is an abnormal cell. As shown in FIG. 9C, when the bright point pattern coincides with the negative pattern, the processing unit 201 determines that the target cell is negative for the measurement item, that is, the target cell is a normal cell.

In a case where the bright point pattern does not coincide with the positive pattern and the negative pattern, the processing unit 201 causes the storage unit 202 to store a result indicating that determination as to the target cell cannot be made for the measurement item. For example, in a case where one cell is determined for two or more measurement items, although the cell is determined to be positive or negative for one of the measurement items, the cell may not be determined for the other of the measurement items. In this case, the processing unit 201 causes the storage unit 202 to store the determination result of the one of the measurement items for the target cell and a result indicating that the target cell has not been determined for the other of the measurement items.

In step S25, the processing unit 201 calculates the number of positive cells, a percentage of positive cells, the number of negative cells, and a percentage of negative cells, for each measurement item, based on the determination result for each cell obtained in step S24. Specifically, the processing unit 201 counts the cells determined as being positive for the target measurement item, to obtain the number N1 of positive cells, and counts the cells determined as being negative for the target measurement item, to obtain the number N2 of negative cells. The processing unit 201 obtains N1/(N1+N2) as the percentage of positive cells and obtains N2/(N1+N2) as the percentage of negative cells.

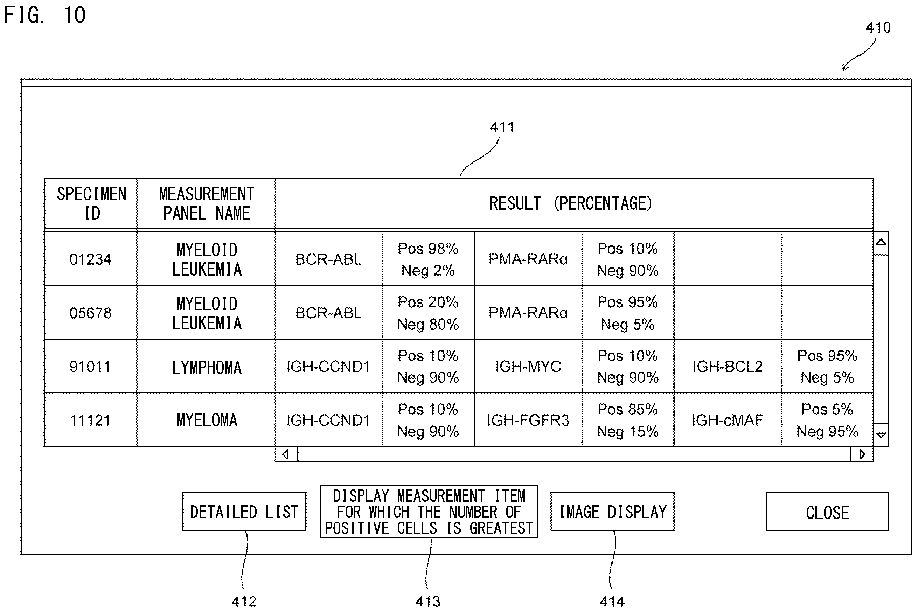

In step S26, the processing unit 201 causes the display unit 203 to display screens 410 to 440 shown in FIG. 10 to FIG. 15 according to display instruction from an operator through the input unit 204. At this time, the processing unit 201 generates the screens 410 to 440, based on the taken images obtained in step S21, the determination result obtained in step S24, and the values calculated in step S25.

As shown in FIG. 10, the screen 410 includes a list 411 and buttons 412 to 414.

In the list 411, a measurement result for each specimen ID is indicated. The display items in the list 411 include the specimen ID, the measurement panel name, and the measurement result for each measurement item. The percentage of positive cells and the percentage of negative cells are displayed by the processing unit 201 as the measurement result for each measurement item, according to the calculation result in step S25. For example, the list 411 illustrated in FIG. 10 indicates that the measurement panel name is "myeloid leukemia" for the specimen ID "01234" in the first line. The specimen is determined for the measurement items "BCR-ABL" and "PMA-RARα", and a percentage of positive cells and a percentage of negative cells for these measurement items are indicated.

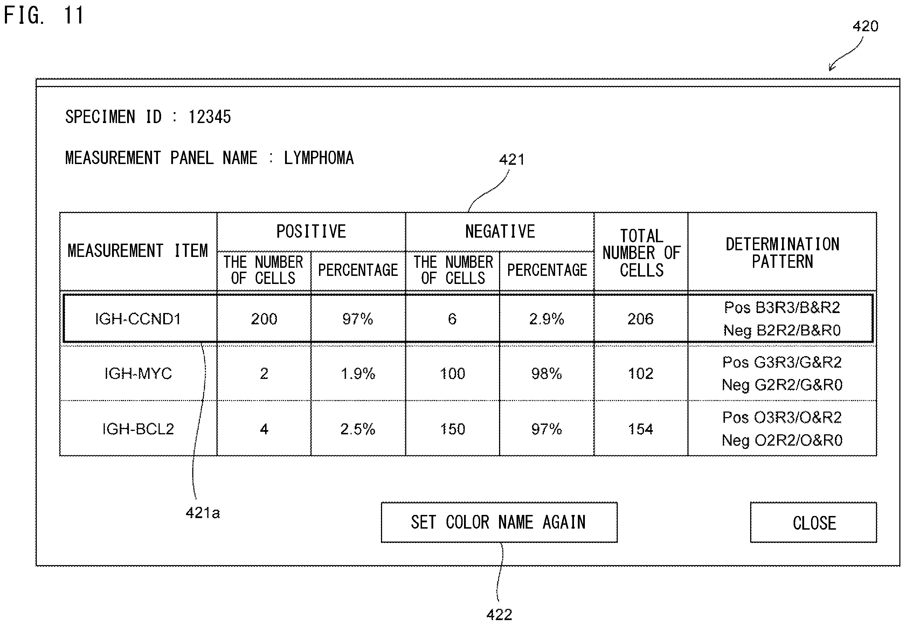

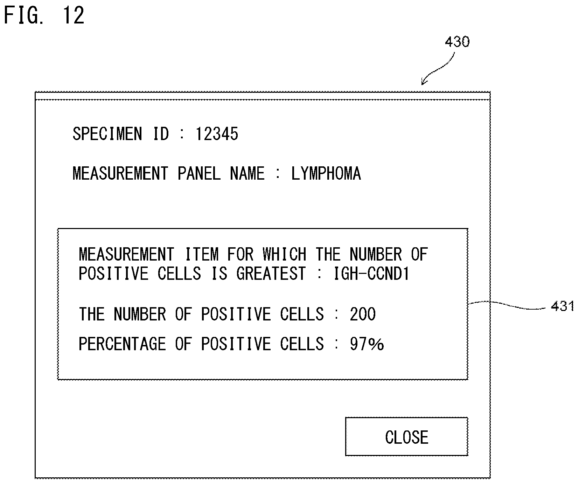

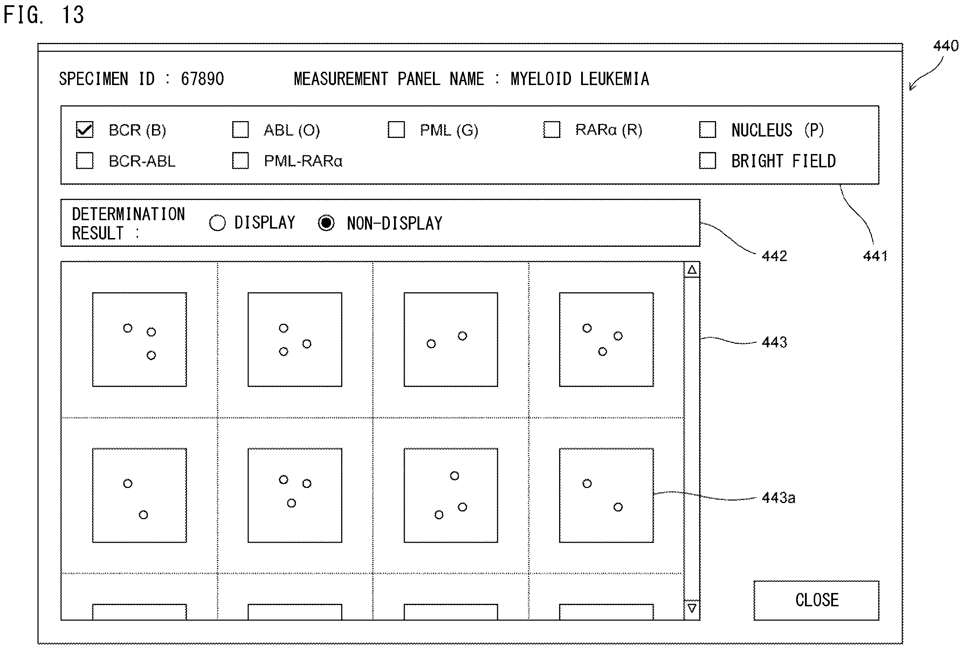

Each line of the list 411 is structured such that the specimen can be selected by operation of an operator. The operator selects one of the specimens indicated in the list 411, and then operates one of the buttons 412 to 414. Thus, the processing unit 201 causes the display unit 203 to display the screen 420, 430, 440 based on the selected specimen. The button 412 is a button for displaying the screen 420 shown in FIG. 11. The button 413 is a button for displaying the screen 430 shown in FIG. 12. The button 414 is a button for displaying the screen 440 shown in FIG. 13 to 15.

As shown in FIG. 11, the screen 420 includes a list 421 and a button 422.

In the list 421, a measurement result for the specimen selected on the screen 410 in FIG. 10 is displayed for each measurement item. The display items of the list 421 include a measurement item, the number of positive cells, a percentage of positive cells, the number of negative cells, a percentage of negative cells, the total number of the cells, and a determination pattern. The number of positive cells, a percentage of positive cells, the number of negative cells, and a percentage of negative cells are displayed by the processing unit 201 based on the calculation result in step S25. The processing unit 201 adds the number of positive cells and the number of negative cells to display the total number of the cells. The contents similar to the positive pattern and the negative pattern displayed in the list 333 in FIG. 6A are displayed, by the processing unit 201, as the determination pattern.

Thus, when the number of positive cells, a percentage of positive cells, the number of negative cells, and a percentage of negative cells are displayed, a doctor and the like are allowed to smoothly determine whether the specimen and the subject are positive or negative for a target measurement item with reference to the displayed information. In the list 421, at least one of the number of positive cells, a percentage of positive cells, the number of negative cells, and a percentage of negative cells may be displayed.

For example, in a case where the percentage of positive cells exceeds a predetermined threshold value, indication representing "possibility of being positive?" for the measurement item so as to indicate that the specimen and the subject may be positive, may be displayed by the processing unit 201. For example, in a case where the percentage of negative cells exceeds a predetermined threshold value, indication representing "possibility of being negative?" for the measurement item so as to indicate that the specimen and the subject may be negative, may be displayed by the processing unit 201. When such a display is performed, a doctor and the like are allowed to smoothly determine whether the specimen and the subject are positive or negative.

The processing unit 201 performs identification display such that an operator is allowed to know the measurement item for which the number of positive cells is greatest or the percentage of the positive cells is highest, among the measurement items displayed in the list 421. Specifically, the processing unit 201 operates so as to add a frame 421a to a line of the measurement item for which the number of positive cells is greatest or the percentage of positive cells is highest. Such an identification display may be performed by using an icon or the like as well as the frame 421a. Thus, the operator is allowed to smoothly obtain information on the measurement item that is prominent due to the number of positive cells being great or a percentage of positive cells being high.

The button 422 is a button for resetting a color name that is information on a color of fluorescence. When an operator operates the button 422, the processing unit 201 closes the screen 420, and causes the display unit 203 to display the input screen 330 shown in FIG. 5A and FIG. 6A. The operator selects a color name on the input screen 330 displayed after the button 422 is operated, as described with reference to FIG. 5A and FIG. 6A, and operates the OK button 334. Thus, the processing unit 201 updates the color name associated with the target substance. By updating the color name, the fluorescence image to be associated with the measurement item is changed, and, therefore, the processing unit 201 performs again the process of steps S23 to S25 in FIG. 8. The screen 410 shown in FIG. 11 is displayed again, by the processing unit 201, based on the process performed again.

Thus, also after the measurement, the operator is allowed to set the color name to be associated with the target substance by the input screen 330 being displayed. Thus, even when the color name inputted before the measurement is incorrect, the operator sets the color name again, thereby obtaining an appropriate measurement result.

As shown in FIG. 12, the screen 430 includes a region 431.

In the region 431, information on the measurement item for which the number of positive cells is greatest or the percentage of the positive cells is highest for the specimen selected on the screen 410 in FIG. 10 is displayed. The display contents in the region 431 include: the measurement item for which the number of positive cells is greatest or the percentage of the positive cells is highest; and the number of positive cells and the percentage of positive cells for the measurement item. The measurement item for which the number of positive cells is greatest, the number of positive cells, and the percentage of positive cells are displayed, by the processing unit 201, based on the calculation result in step S25. Also in this case, similarly to the frame 421a in FIG. 11, the operator is allowed to smoothly obtain information on the measurement item that is prominent due to the number of positive cells being great or a percentage of positive cells being high.

As shown in FIGS. 13 to 15, the screen 440 includes display setting regions 441 and 442 and an image display region 443.

The display setting region 441 includes selection portions for selection of an image to be displayed in the image display region 443 for a specimen selected on the screen 410 in FIG. 10. The selection portions in the display setting region 441 include check boxes. The operator is allowed to select one or more selection portions from among the selection portions in the display setting region 441. The selection portions in the display setting region 441 are provided so as to correspond to the measurement items set for a target specimen. For example, in the example shown in FIG. 13 to 15, since the measurement items BCR-ABL and PML-RARα are set for a specimen, the selection portions corresponding to BCR gene, ABL gene, PML gene, RARα gene, and a nucleus which are the target substances, and the selection portions corresponding to the measurement items BCR-ABL and PML-RARα are provided in the display setting region 441. The selection portion corresponding to the bright field image is also provided in the display setting region 441.

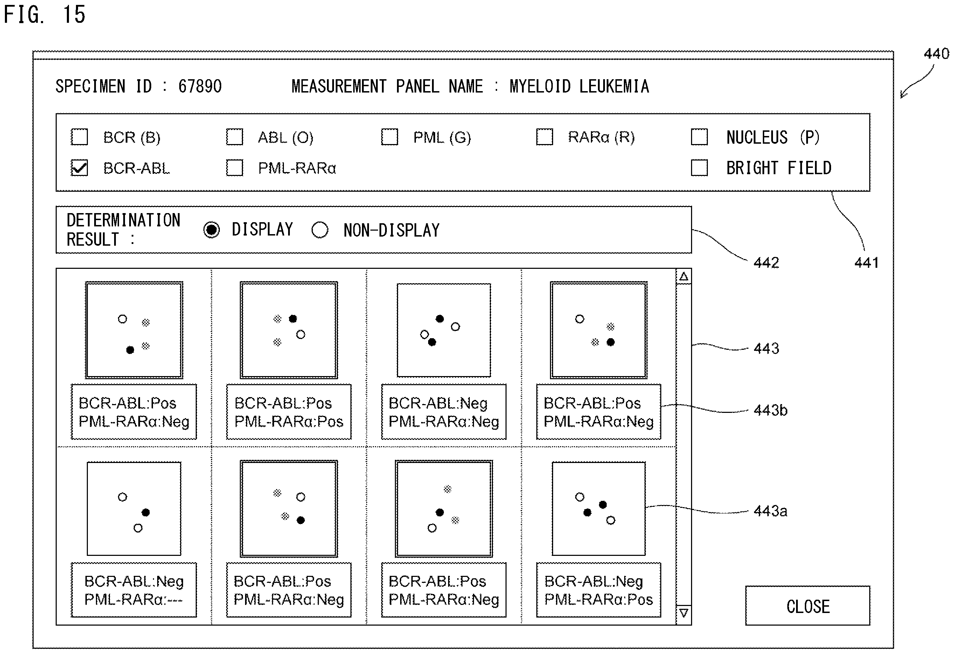

The display setting region 442 includes a selection portion for selection and determination as to whether a label 443b indicating a determination result for a cell corresponding to an image 443a is to be displayed below the image 443a displayed in the image display region 443. In the label 443b, a positive or negative determination result for each measurement item is displayed as shown in FIG. 15. The selection portion in the display setting region 442 is implemented by a radio button. The operator is allowed to select one of the selection portion corresponding to "display" and the selection portion corresponding to "non-display".

In the image display region 443, the image 443a for the specimen selected in the screen 410 in FIG. 10 is displayed. The image 443a for the target specimen is displayed, by the processing unit 201, in the image display region 443 based on the item designated by the selection portion in the display setting region 441. In a case where the item designated by the selection portion in the display setting region 442 is "display", the label 443b representing the determination result is displayed, by the processing unit 201, below the image 443a.

In the example shown in FIG. 13, BCR gene is selected in the display setting region 441, and non-display is selected in the display setting region 442. Therefore, the processing unit 201 operates to display the fluorescence image corresponding to BCR gene, as the image 443a, in the image display region 443, and operates so as not to display the label 443b below the image 443a. In a case where the fluorescence image of the gene is displayed, the processing unit 201 reads a gray scale fluorescence image from the storage unit 202, and converts the color of the read fluorescence image to a color corresponding to the color name, to display the obtained image in the image display region 443. In the example shown in FIG. 13, the color name of the BCR gene is set as blue, and, therefore, the processing unit 201 converts, to blue, the color of the fluorescence image read from the storage unit 202, and displays the obtained image.

Thus, when the fluorescence image, based on the target substance, taken by the imaging unit 154 is displayed in the image display region 443, the operator is allowed to confirm the location of the target substance, presence or absence of the target substance, an amplified state of the target substance, and the like, with reference to the fluorescence image. Thus, the operator is allowed to determine, for example, whether a cell including the target substance is positive or negative.

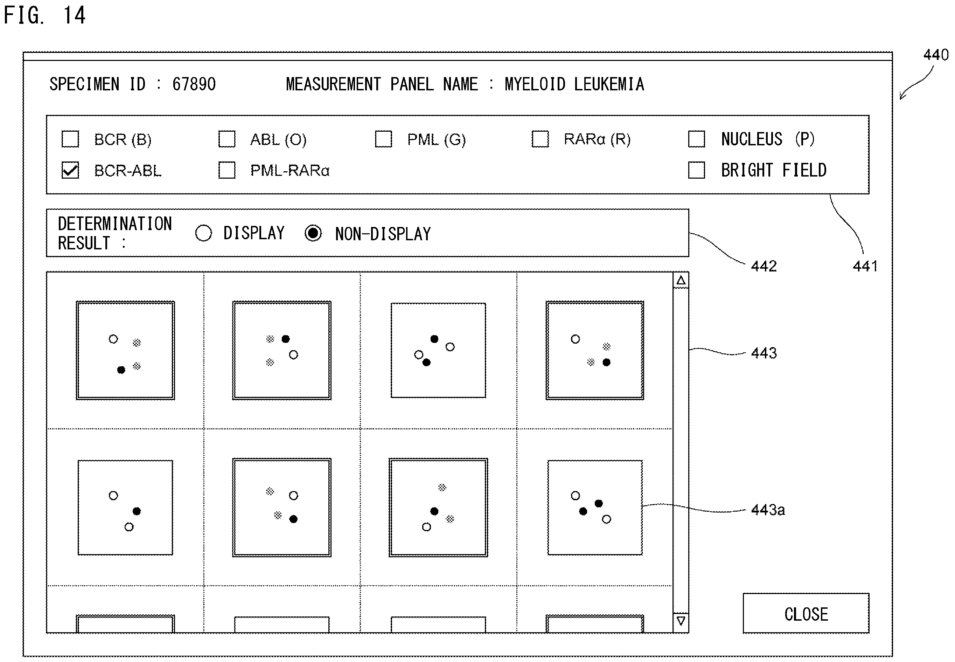

In the example shown in FIG. 14, the measurement item "BCR-ABL" is selected in the display setting region 441, and "non-display" is selected in the display setting region 442. Therefore, an image obtained by the fluorescence images of the BCR gene and the ABL gene related to the measurement item BCR-ABL being combined with each other is displayed, by the processing unit 201, as the image 443a in the image display region 443. In a case where fluorescence images of a plurality of kinds of related genes are combined as are used for the measurement item BCR-ABL, the processing unit 201 converts the color of each fluorescence image to a color according to the color setting, and further adjusts, as appropriate, the colors of the plurality of fluorescence images in which the colors have been converted, to combine the obtained images.

In the example shown in FIG. 14, the color name of the BCR gene is set as blue, and the color name of the ABL gene is set as orange, and, therefore, the processing unit 201 converts, to blue, the color of the fluorescence image of the BCR gene which is read from the storage unit 202 and converts, to orange, the color of the fluorescence image of the ABL gene which is read from the storage unit 202. The processing unit 201 adjusts, as appropriate, the two fluorescence images in which the colors have been converted, and combines the two images, and displays the obtained image.

Thus, when the composite image is displayed in the image display region 443, the operator is allowed to smoothly confirm locations of a plurality of kinds of target substances with reference to the composite image. Thus, the operator is allowed to determine, for example, whether the cell including the target substance is positive or negative.

In a case where the measurement item is designated in the display setting region 441, the processing unit 201 performs identification display so as to allow an operator to know whether the determination result of the measurement item is positive or negative, for the image 443a displayed in the image display region 443. Specifically, in a case where the determination result of the measurement item designated in the display setting region 441 is positive, the outer frame of the image 443a is rendered as double lines by the processing unit 201 as shown in FIG. 14. Thus, the operator is allowed to smoothly know whether or not the cell indicated in the image 443a is positive for the measurement item selected in the display setting region 441.

In the example shown in FIG. 15, the measurement item "BCR-ABL" is selected in the display setting region 441, and "display" is selected in the display setting region 442. Therefore, the processing unit 201 operates to display an image obtained by fluorescence images of the BCR gene and the ABL gene being combined with each other, as the image 443a, in the image display region 443, and display the label 443b below each image 443a. In this case, for example, when determination as to the measurement items "BCR-ABL" and "PML-RARα" is performed in step S24 in FIG. 8, the determination result of the measurement item "BCR-ABL" and the determination result of the measurement item "PML-RARα" are displayed in the labels 443b. In the label 443b, "Pos" indicates that the cell is positive for the measurement item, and "Neg" indicates that the cell is negative for the measurement item.

Thus, when both the image 443a of each cell and the label 443b are displayed, the operator is allowed to refer to the image 443a and confirm, for each cell, the results of the determinations which have been performed for all the measurement items for the cell.

In a case where, when whether the cell is positive or negative is determined, the bright point pattern does not coincide with the positive pattern and the negative pattern, information indicating that the determination cannot be made is displayed in the label 443b by the processing unit 201. In the example shown in FIG. 15, "---" is displayed as in the label 443b, for the cell, displayed in the lower left portion of the image display region 443 since determination for measurement item "PML-RARα" cannot be made. Instead of "---", "excluded" representing exclusion may be displayed.

When the screens 410, 420, 430, and 440 as described are displayed on the display unit 203, a doctor and the like are allowed to confirm the measurement result in detail with reference to the screens. The doctor and the like can utilize the displayed contents on the screens for determining whether the specimen is positive or negative.

### <Embodiment 2>

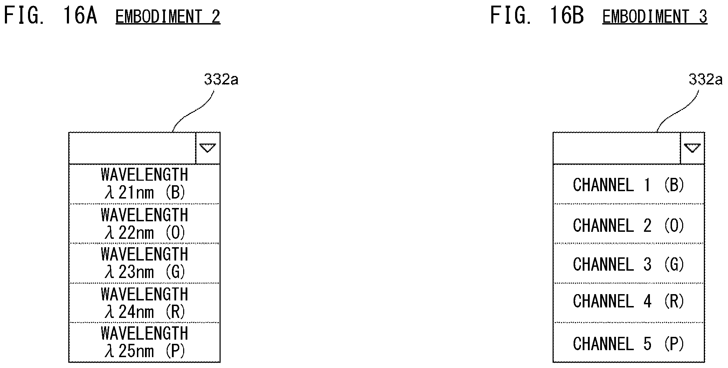

In Embodiment 1, information, on a color of fluorescence, set in the selection portion 332a is a color name of a color of fluorescence incident on each of the regions 161 to 165 of the imaging unit 154 as shown in FIG. 5B, in the list 332 of the input screen 330 shown in FIG. 5A. Meanwhile, in Embodiment 2, information, on a color of fluorescence, set in the selection portion 332a is a wavelength band of the fluorescence incident on each of the regions 161 to 165 of the imaging unit 154 as shown in FIG. 16A. The other configurations of Embodiment 2 are the same as described for Embodiment 1.

In Embodiment 2, in a case where the wavelengths λ21, λ22, λ23, λ24, and λ25 are selected in the selection portions 332a, the processing unit 201 performs processing similar to the processing performed when blue, orange, green, red, and purple are selected in Embodiment 1. Thus, also in Embodiment 2, the processing unit 201 can obtain a color of fluorescence generated from a target substance, thereby appropriately analyzing the target substance based on the inputted information on the color of the fluorescence, and the fluorescence image.

### <Embodiment 3>

In Embodiment 3, the information, on colors of fluorescences, set in the selection portion 332a is the channels 1 to 5 as shown in FIG. 16B. The channels 1 to 5 form a system for performing processing based on light incident on the regions 161 to 165 of the imaging unit 154 as described with reference to FIG. 2. The other configurations of Embodiment 3 are the same as described for Embodiment 1.

Also in Embodiment 3, in a case where the channels 1 to 5 are selected in selection portions 332a, the processing unit 201 performs processing similar to the processing performed when blue, orange, green, red, and purple are selected in Embodiment 1. Thus, also in Embodiment 3, the processing unit 201 can obtain a color of fluorescence generated from a target substance, thereby appropriately analyzing the target substance.

### <Embodiment 4>

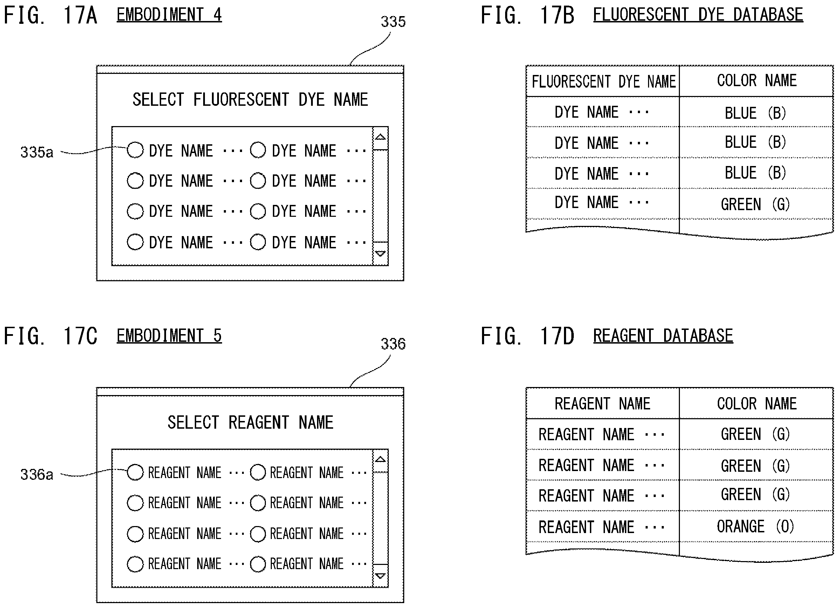

In Embodiment 4, information, on a color of fluorescence, set by the selection portion 332a is a fluorescent dye name. In Embodiment 4, when the selection portion 332a is operated in the list 332 shown in FIG. 5A, the processing unit 201 causes the display unit 203 to display a screen 335 shown in FIG. 17A. The fluorescent dye names are displayed on the screen 335, by the processing unit 201, based on the fluorescent dye names registered in a fluorescent dye database shown in FIG. 17B. The screen 335 includes a plurality of selection portions 335a corresponding to the fluorescent dye names. When an operator operates the selection portion 335a corresponding to a target fluorescent dye name, the processing unit 201 operates so as to close the screen 335 and display the fluorescent dye name selected on the screen 335, in the selection portion 332a of the list 332. The operator operates the selection portion 332a for each target substance, to display the screen 335, and inputs the fluorescent dye name for each target substance through the screen 335.

As shown in FIG. 17B, in Embodiment 4, the storage unit 202 stores the fluorescent dye database. In the fluorescent dye database, a plurality of combinations each including a fluorescent dye name of a fluorescent dye which may be used, and a color of fluorescence corresponding to the fluorescent dye name, are registered in advance. When the OK button 334 is operated on the input screen 330 shown in FIG. 5A, the processing unit 201 obtains the color of fluorescence corresponding to the fluorescent dye name displayed in the selection portion 332a, according to the fluorescent dye database. Thereafter, the processing unit 201 performs the same process as in Embodiment 1, based on the obtained color of fluorescence. The other configurations of Embodiment 4 are the same as described for Embodiment 1.

Also in Embodiment 4, similarly to Embodiment 1, the processing unit 201 can obtain a color of fluorescence generated from a target substance, thereby appropriately analyzing the target substance. In Embodiment 4, an operator merely inputs a name of a fluorescent dye used for labeling a target substance without knowing, for example, a color or a wavelength of the fluorescence the image of which is taken by the imaging unit 154, thereby easily associating the target substance with information on the color of the fluorescence.

### <Embodiment 5>

In Embodiment 5, information, on a color of fluorescence, set by the selection portion 332a is a reagent name. The reagent name is a name of a reagent for labeling a predetermined target substance with a fluorescent dye. In Embodiment 5, when the selection portion 332a is operated in the list 332 shown in FIG. 5A, the processing unit 201 causes the display unit 203 to display a screen 336 shown in FIG. 17C. The reagent name is displayed on the screen 336, by the processing unit 201, based on the reagent name registered in a reagent database shown in FIG. 17D. The screen 336 includes a plurality of selection portions 336a corresponding to the reagent names. When an operator operates the selection portion 336a corresponding to a target reagent name, the processing unit 201 operates so as to close the screen 336 and display the reagent name selected on the screen 336, in the selection portion 332a of the list 332. The operator operates the selection portion 332a for each target substance, to display the screen 336, and inputs a reagent name for each target substance through the screen 336.

As shown in FIG. 17D, in Embodiment 5, the storage unit 202 stores the reagent database. In the reagent database, a plurality of combinations each including a reagent name of a reagent which may be used, and a color of fluorescence generated from a fluorescent dye corresponding to the reagent name, are registered in advance. When the OK button 334 is operated on the input screen 330 in FIG. 5A, the processing unit 201 obtains the color of fluorescence corresponding to the reagent name displayed in the selection portion 332a, according to the reagent database. Thereafter, the processing unit 201 performs the same process as in Embodiment 1, based on the obtained color of fluorescence. The other configurations of Embodiment 5 are the same as described for Embodiment 1.

Also in Embodiment 5, similarly to Embodiment 1, the processing unit 201 can obtain a color of fluorescence generated from a target substance, thereby appropriately analyzing the target substance. In Embodiment 5, an operator merely inputs a name of a reagent used for labeling a target substance without knowing, for example, a color or a wavelength of the fluorescence the image of which is taken by the imaging unit 154, thereby easily associating the target substance with the information on the color of the fluorescence.

### <Embodiment 6>

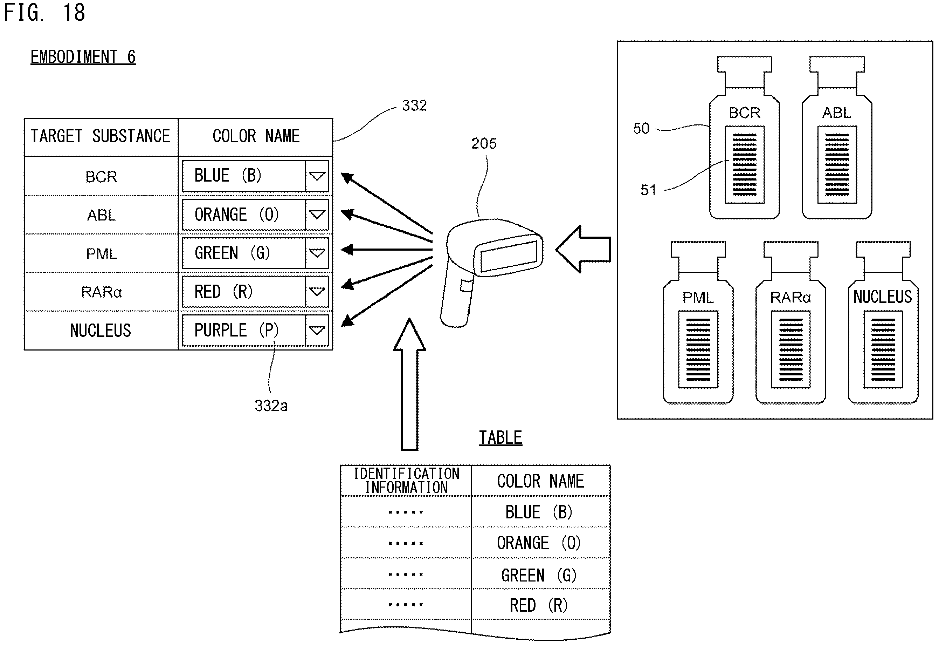

As shown in FIG. 18, in Embodiment 6, a plurality of reagent containers 50 each contain a reagent for labeling a target substance with a fluorescent dye. To each reagent container 50, a label having identification information 51 as information is adhered. The identification information 51 includes barcode information. As shown in FIG. 18, the storage unit 202 stores a table in which a color name is registered as information on a color of fluorescence so as to correspond to the identification information 51. The identification information 51 is set so as to correspond to a color of fluorescence generated from a fluorescent dye in the reagent contained in each reagent container 50, and the identification information 51 of the reagent container 50 is different for each color of fluorescence generated from the fluorescent dye in the reagent contained in the reagent container 50. In Embodiment 6, the analyzing unit 200 includes an information obtaining unit 205. The information obtaining unit 205 obtains the identification information 51, and is implemented by a barcode reader.

The processing unit 201 receives input of information on a color of fluorescence, based on the identification information 51 obtained by the information obtaining unit 205. Specifically, based on the identification information 51 obtained by information obtaining unit 205, the processing unit 201 obtains information on a color of fluorescence corresponding to the identification information 51, from the table shown in FIG. 18. Thus, the processing unit 201 obtains the information on the color of the fluorescence generated from the fluorescent dye in the reagent contained in the reagent container 50. The other configurations of Embodiment 6 are the same as described for Embodiment 1.

The identification information 51 may be implemented by a RFID, and the information obtaining unit 205 may be implemented by an antenna for reading the RFID from a RFID tag. The identification information 51 may be implemented by a particular structure such as a cut or a hole, and the information obtaining unit 205 may be implemented by a device for identifying the particular structure such as a cut or a hole.

In the example shown in FIG. 18, the reagent containers 50 contain a reagent for labeling BCR gene, a reagent for labeling ABL gene, a reagent for labeling PML gene, a reagent for labeling RARα gene, and a reagent for labeling a nucleus, respectively. The identification information 51 is different according to the reagent contained in the reagent container 50.

An operator operates the selection portion 332a on which input is performed, and information is then read from the identification information 51 by using the information obtaining unit 205, whereby the processing unit 201 reads information on a color of fluorescence corresponding to the identification information 51, according to the table stored in the storage unit 202. The processing unit 201 sets the color name in the corresponding selection portion 332a, as shown in FIG. 18. Thus, as in a case where an operator operates the selection portion 332a to set a color name in Embodiment 1, a color name corresponding to the target substance is set in the selection portion 332a.

Also in Embodiment 6, similarly to Embodiment 1, the processing unit 201 can obtain a color of fluorescence generated from a target substance, thereby appropriately analyzing the target substance. In Embodiment 6, an operator is allowed to smoothly input information on a color of fluorescence so as to associate the information on the color of fluorescence with a target substance, by information being read from the identification information 51, with the use of the information obtaining unit 205. An operator merely reads information from the identification information 51 by means of the information obtaining unit 205 without knowing, for example, a color or a wavelength of fluorescence the image of which is taken by the imaging unit 154, thereby easily associating the target substance with information on the color of the fluorescence.

In the table shown in FIG. 18, the target substance name may be further registered so as to correspond to the identification information. In this case, the processing unit 201 can obtain a target substance name as well as a color name based on the identification information 51 obtained by the information obtaining unit 205, whereby an operator need not specify a target substance by operating the selection portion 332a. As the identification information 51, information on a color of fluorescence may be stored, or information on a color of fluorescence and a target substance name may be stored. In this case, the table shown in FIG. 18 need not be used.

### <Embodiment 7>

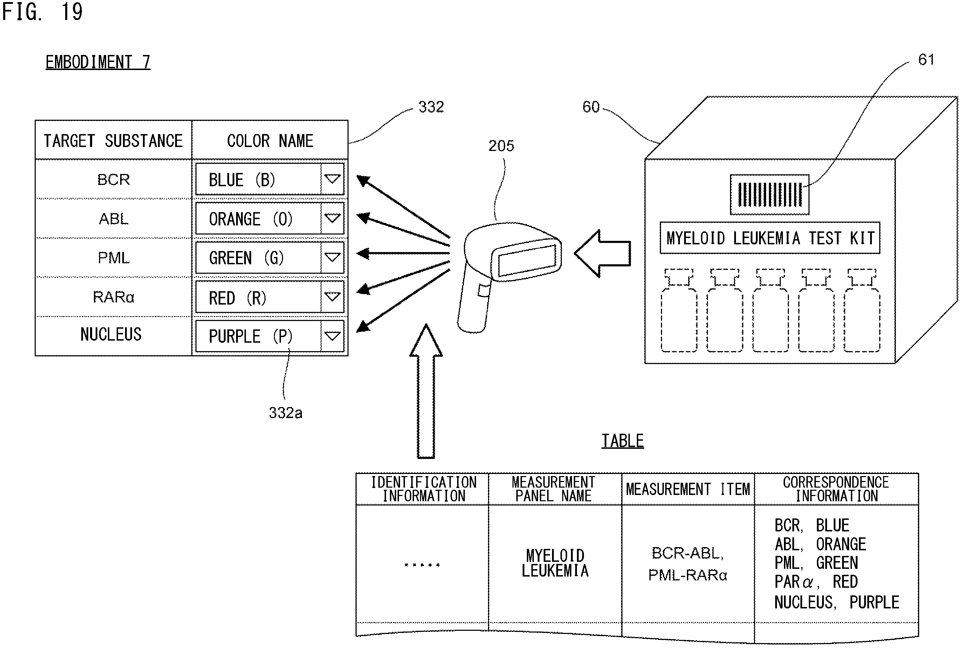

As shown in FIG. 19, in Embodiment 7, a test kit container 60 contains reagent containers used for measurement items corresponding to a measurement panel name. The reagent containers in the test kit container 60 contain reagents for labeling target substances with fluorescent dyes. To the test kit container 60, a label having identification information 61 as information is adhered. The identification information 61 includes barcode information. As shown in FIG. 19, the storage unit 202 stores a table in which a measurement panel name, measurement items, and correspondence information indicating correspondence between target substances and information on colors of fluorescences are registered so as to correspond to the identification information 61. That is, the identification information 61 is set so as to correspond to kinds of reagents in the test kit container 60, and the identification information 61 of the test kit container 60 is different for each kind of the reagent contained in the test kit container 60.

Based on the identification information 61 obtained by the information obtaining unit 205, the processing unit 201 obtains a measurement panel name, a measurement item, and correspondence information which correspond to the identification information 61, from the table shown in FIG. 19. Thus, the processing unit obtains information on a color of fluorescence generated from a fluorescent dye in the reagent contained in the reagent container. The other configurations of Embodiment 7 are the same as described for Embodiment 6.

The identification information 61 may be implemented by a RFID tag, or a particular structure such as a cut or a hole, similarly to the identification information 51 in Embodiment 6.

In the example shown in FIG. 19, the test kit container 60 contains five reagent containers for a case where the measurement panel name is "myeloid leukemia". The five reagent containers in the test kit container 60 contain a reagent for labeling BCR gene, a reagent for labeling ABL gene, a reagent for labeling PML gene, a reagent for labeling RARα gene, and a reagent for labeling a nucleus, respectively.

An operator operates to read information from the identification information 61 by using the information obtaining unit 205, whereby the processing unit 201 reads information on a color of fluorescence corresponding to the identification information 61, according to the table stored in the storage unit 202. The processing unit 201 sets a color name in the selection portion 332a in the list 332, as shown in FIG. 19. Thus, similarly to Embodiment 1 in which an operator operates the selection portion 332a to set a color name, a color name corresponding to a target substance is set in the selection portion 332a.

In this case, the processing unit 201 receives the measurement panel name and the measurement item as well as information on the color of the fluorescence. Thus, the operator need not select the measurement panel name through the screen 310 shown in FIG. 4B, and need not select the measurement item through the screen 320 shown in FIG. 4C.

Also in Embodiment 7, similarly to Embodiment 1, the processing unit 201 can obtain a color of fluorescence generated from a target substance, thereby appropriately analyzing the target substance. In Embodiment 7, an operator operates to read information from the identification information 61 once by using the information obtaining unit 205, whereby information on colors of fluorescences can be smoothly and quickly inputted so as to correspond to a plurality of kinds of target substances. Similarly to Embodiment 6, an operator merely reads the information from the identification information 61 by means of the information obtaining unit 205, thereby easily associating a target substance related to a measurement item, with information on a color of fluorescence.

In the identification information 61, the measurement panel name, the measurement items, and the correspondence information may be stored. In this case, the table shown in FIG. 19 need not be used.

In embodiments 6 and 7, color names are stored as information on colors of fluorescences for the identification information 51, 61. However, as in Embodiments 2 to 5, wavelength bands of fluorescences, channels, fluorescent dye names, reagent names, or the like may be stored.

### <Embodiment 8>

In Embodiment 1, information on a color of fluorescence corresponding to a target substance is inputted through the input screen 330 in step S13 shown in FIG. 4A. That is, in Embodiment 1, an operator operates the selection portion 332a to select a color name, whereby information on a color of fluorescence is set so as to correspond to a target substance such that the information is variable. However, information on a color of fluorescence corresponding to a target substance may be automatically set by the processing unit 201.

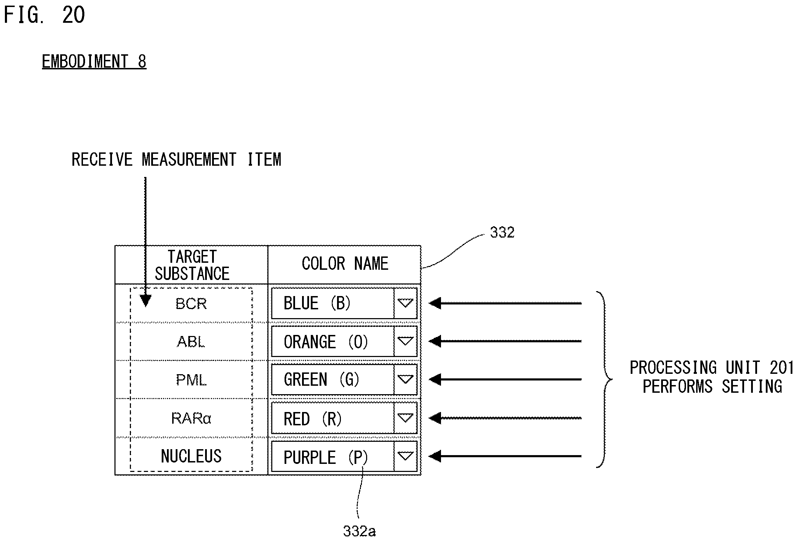

As shown in FIG. 20, in Embodiment 8, when the processing unit 201 receives input of a measurement item through the screen 320 shown in FIG. 4C, the processing unit 201 sets, in the list 332 of the input screen 330, information on colors of fluorescences so as to associate the information on the colors of fluorescences with a plurality of target substances related to the received measurement item.

For example, as illustrated in FIG. 20, when the processing unit 201 receives input of BCR-ABL and PML-RARα as the measurement items, blue, orange, green, red, and purple are automatically set for BCR gene, ABL gene, PML gene, RARα gene, and nucleus, respectively. Thus, in Embodiment 8, when an operator inputs the measurement item, the processing unit 201 automatically sets the information on the colors of fluorescences, in the selection portions 332a corresponding to the target substances related to the measurement item. Thus, the operator need not set the information on the color of the fluorescence for the target substance. The processing unit 201 sets the information on the color of the fluorescence for the target substance, whereby the operator can be prevented from making erroneous setting.

The processing unit 201 may receive input of a plurality of target substances instead of a measurement item. In this case, the processing unit 201 sets information on colors of fluorescences so as to associate the information on the colors of fluorescences with the plurality of target substances having been received through the input.

In a case where an operator operates a part of the selection portions 332a in the input screen 330 to set information on colors of fluorescences, the processing unit 201 may automatically set information on colors of fluorescences for the remaining selection portions 332a. Thus, the operator need not set the information on the colors of the fluorescences for all the target substances. The processing unit 201 sets the information on the colors of the fluorescences for a part of the target substances, thereby preventing the information on the colors of the fluorescences for the part of the target substances from being erroneously set.

The selection portion 332a in which information on a color of fluorescence has been set by the processing unit 201, may be structured so as not to allow an operator to perform resetting, or may be structured so as to allow an operator to perform resetting. In a case where information, on a color of fluorescence, set by the processing unit 201 cannot be changed by an operator, the operator can be prevented from making erroneous setting. Meanwhile, in a case where information, on a color of fluorescence, set by the processing unit 201 can be changed by an operator, the operator is allowed to freely change a color name after confirming the color name set by the processing unit 201.

### <Example 1>

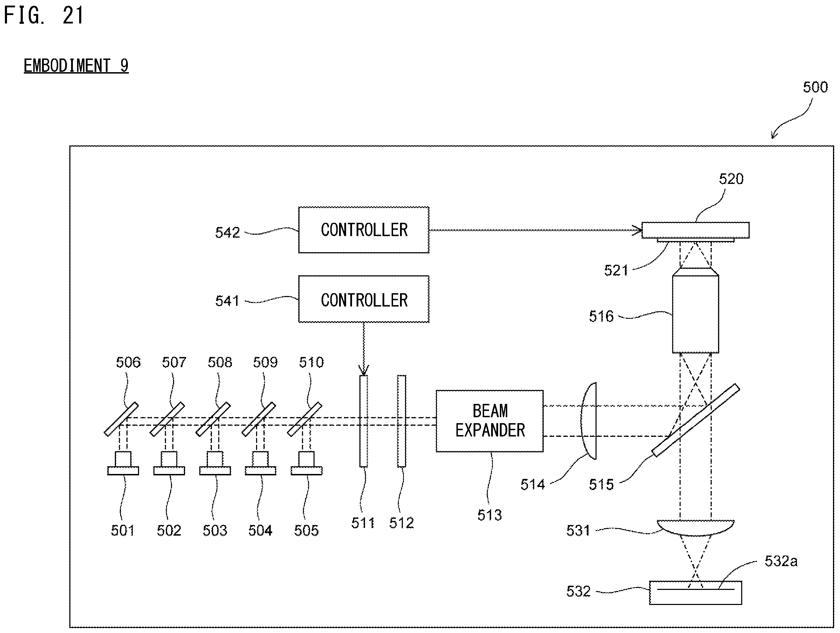

As shown in FIG. 21, in Example 1 not forming part of the present invention, the sample analyzer 10 includes a measurement unit 500 having a fluorescence microscope instead of the measurement unit 100 shown in FIG. 1. The other components of Example 1 are the same as the components shown in FIG. 1.

The measurement unit 500 includes light sources 501 to 505, a mirror 506, dichroic mirrors 507 to 510, a shutter 511, a 1/4 wave plate 512, a beam expander 513, a condenser lens 514, a dichroic mirror 515, an object lens 516, a stage 520, a condenser lens 531, an imaging unit 532, and controllers 541 and 542. On the stage 520, a glass slide 521 is placed. On the glass slide 521, a measurement specimen 21 is placed.

The light sources 501 to 505 are similar to the light sources 121 to 125, respectively, shown in FIG. 1. The mirror 506 reflects light from the light source 501. The dichroic mirror 507 allows light from the light source 501 to pass therethrough, and reflects light from the light source 502. The dichroic mirror 508 allows light from the light sources 501 and 502 to pass therethrough, and reflects light from the light source 503. The dichroic mirror 509 allows light from the light sources 501 to 503 to pass therethrough, and reflects light from the light source 504. The dichroic mirror 510 allows light from the light sources 501 to 504 to pass therethrough, and reflects light from the light source 505. The optical axes of light from the light sources 501 to 505 are made coaxial with each other by the mirror 506 and the dichroic mirrors 507 to 510.

The shutter 511 is driven by the controller 541, and switches between a state where light emitted from the light sources 501 to 505 is caused to pass therethrough, and a state where light emitted from the light sources 501 to 505 is blocked. Thus, a time during which light is applied to the measurement specimen 21 is adjusted. The 1/4 wave plate 512 converts linearly polarized light emitted from the light sources 501 to 505 into circularly polarized light. A fluorescent dye reacts to light in a predetermined polarization direction. Therefore, light, for excitation, emitted from the light sources 501 to 505 is converted into circularly polarized light, whereby the polarization direction of the light for excitation is likely to be aligned with the polarization direction in which the fluorescent dye reacts. Thus, the fluorescent dye can be efficiently excited to emit fluorescence. The beam expander 513 widens the light irradiation region on the glass slide 521. The condenser lens 514 collects light such that collimated light is applied from the object lens 516 to the glass slide 521.

The dichroic mirror 515 reflects light emitted from the light sources 501 to 505, and allows fluorescence generated from the measurement specimen 21 to pass therethrough. The object lens 516 guides the light reflected by the dichroic mirror 515 to the glass slide 521. The stage 520 is driven by the controller 542. The fluorescence generated from the measurement specimen 21 passes through the object lens 516 and passes through the dichroic mirror 515. The condenser lens 531 collects the fluorescence that has passed through the dichroic mirror 515, and guides the fluorescence to a light receiving surface 532a of the imaging unit 532. The imaging unit 532 takes an image of the fluorescence applied to the light receiving surface 532a, and generates a fluorescence image. The imaging unit 532 is implemented by, for example, a CCD or the like.

The controllers 541 and 542 and the imaging unit 532 are connected to the processing unit 201 shown in FIG. 1, and the processing unit 201 controls the controllers 541 and 542, and the imaging unit 532, and receives the fluorescence image taken by the imaging unit 532. In the fluorescence image taken by the imaging unit 532, cells may be densely packed, unlike in a case where the flow cell 110 is used as shown in FIG. 1. Therefore, the processing unit 201 performs, for example, a process of dividing the obtained fluorescence image for each cell nucleus, or a process of setting a region corresponding to one cell nucleus in the fluorescence image.

Also in Example 1, the processing unit 201 receives input of information on a color of fluorescence so as to correspond to a target substance through the input screen 330 shown in FIG. 5A. Thus, similarly to Embodiment 1, the processing unit 201 can obtain a color of fluorescence generated from a target substance, thereby appropriately analyzing the target substance. When, as in Embodiment 1, the measurement specimen 21 is caused to flow through the flow cell 110 according to the flow cytometry, and the fluorescence image is obtained for each cell, a large amount of the measurement specimen 21 can be measured, a large amount of taken images can be quickly obtained, and smooth analysis of the target substance can be automatically performed.

### <Other embodiments>

In Embodiments 1 to 8, fluorescence detected by the measurement unit 100 is fluorescence generated from a fluorescent dye for labelling a target substance. However, the fluorescence may be intrinsic fluorescence generated from a target substance. Although images of a plurality of kinds of fluorescences having different wavelengths are taken by the same imaging unit 154 in the measurement unit 100, imaging units different for each wavelength may be used to perform detection.

In Embodiments 1 to 8, the imaging unit 154 takes an image of fluorescence and generates a fluorescence image, and the processing unit 201 analyzes a target substance based on the generated fluorescence image. However, in a case where analysis is performed based on an intensity of fluorescence, a light detector may be used instead of the imaging unit 154. That is, the processing unit 201 may perform analysis by using detection result from the light detector as well as the detection result by the imaging unit. In this case, the light detector receives fluorescence, and outputs a signal based on an intensity of the fluorescence, and the processing unit 201 analyzes a target substance according to the signal based on the intensity of the fluorescence.

In Embodiments 1 to 8, whether genetic translocation is positive or negative for a measurement item related to genetic translocation is determined. However, whether gene amplification, deletion, inversion, or the like is positive or negative for a measurement item related thereto may be determined. Also in this case, the processing unit 201 receives input of information on a color of fluorescence for each target substance through a screen similar to the input screen 330 shown in FIG. 5A. The processing unit 201 extracts fluorescence and a fluorescence region of a nucleus from a fluorescence image based on a gene related to the measurement item, and determines whether the result is positive or negative for the measurement item, based the extracted fluorescence region.

### DESCRIPTION OF THE REFERENCE CHARACTERS

10 sample analyzer
21 measurement specimen
51, 61 identification information
100, 500 measurement unit
110 flow cell
121 to 126
light source
154 imaging unit
201 processing unit
202 storage unit
203 display unit
205 information obtaining unit
330 input screen

## Claims

1. A sample analyzer (10) for analyzing a measurement specimen (21) containing a plurality of kinds of target substances each labeled with a fluorescent dye, the sample analyzer (10) comprising:
a measurement unit (100) comprising:
a flow cell (110) allowing the measurement specimen (21) to flow therethrough;
a light source (121, ... 126) configured to apply light (λ11, ... λ16) to the measurement specimen (21) flowing through the flow cell (110) to generate, from the fluorescent dye, a plurality of kinds of fluorescences (λ21, ... λ25) having different wavelengths;
an imaging unit (154) configured to take an image of the fluorescence (λ21, ... λ25) generated from a cell in the measurement specimen (21) flowing through the flow cell (110), for each color of the fluorescence (λ21, ... λ25), and generate a fluorescence image; and
a processing unit (201) configured to analyze the target substances based on the fluorescence image and information on colors of fluorescences (λ21, ... λ25), which is set to be variable so as to correspond to the plurality of kinds of target substances,
wherein the analyzing the target substances comprises:
receiving information on colors of fluorescences (λ21, ... λ25) corresponding to the respective target substances;
obtaining a positive pattern and a negative pattern based on the information on colors of fluorescences (λ21, ... λ25), the positive pattern and the negative pattern being bright point patterns;
extracting a fluorescence region from the fluorescence image and obtaining a bright point pattern of the cell based on the fluorescence region and the information on colors of fluorescences (λ21, ... λ25); and
determining whether the cell is positive or negative by comparing the bright point pattern of the cell with the positive pattern and the negative pattern.

2. The sample analyzer (10) of claim 1, comprising a display unit (203), wherein
the processing unit (201) causes the display unit (203) to display an input screen for receiving input of the information on the color of the fluorescence (λ21, ... λ25) such that the information on the color of the fluorescence (λ21, ... λ25) corresponds to the target substance.

3. The sample analyzer (10) of claim 2, wherein the input screen comprises a plurality of sets each including an item for the target substance and an item for inputting the information on the color of the fluorescence (λ21, ... λ25) .

4. The sample analyzer (10) of claim 2 or 3, wherein
the target substance is related to the measurement item,
the processing unit (201) receives input of the measurement item, and
the input screen comprises a set of an item for the target substance corresponding to the inputted measurement item, and an item for inputting the information on the color of the fluorescence (λ21, ... λ25) .

5. The sample analyzer (10) of any one of claims 1 to 4, comprising an information obtaining unit (205) configured to obtain identification information, wherein
the processing unit (201) receives input of the information on the color of the fluorescence (λ21, ... λ25), based on the identification information obtained by the information obtaining unit (205).

6. The sample analyzer (10) of claim 5, comprising a storage unit (202) configured to store the information on the color of the fluorescence (λ21, ... λ25) such that the information on the color of the fluorescence (λ21, ... λ25) corresponds to the identification information, wherein
the processing unit (201) receives input of the information on the color of the fluorescence (λ21, ... λ25) which corresponds to the target substance by reading, from the storage unit (202), the information, on the color of the fluorescence (λ21, ... λ25), which corresponds to the identification information obtained by the information obtaining unit (205).

7. The sample analyzer (10) of claim 6, wherein
the storage unit (202) stores the plurality of kinds of target substances related to the measurement item such that the plurality of kinds of target substances correspond to the identification information, and
the processing unit (201) receives input of the information on the colors of the fluorescences (λ21, ... λ25) which correspond to the plurality of kinds of target substances related to the measurement item, by reading, from the storage unit (202), the measurement item which corresponds to the identification information obtained by the information obtaining unit (205), and the information, on the colors of the fluorescences (λ21, ... λ25), corresponding to the identification information.

8. The sample analyzer (10) of any one of claims 1 to 7, wherein
the information on the color of the fluorescence (λ21, ... λ25) represents at least one of a color name of the fluorescence, a wavelength of the fluorescence (λ21, ... λ25), a kind of fluorescence labeling, and a kind of a reagent based on the fluorescence labeling.

9. The sample analyzer (10) of any one of claims 1 to 8, comprising a display unit (203), wherein
the processing unit (201) causes the display unit (203) to display the fluorescence image taken by the imaging unit (154) .

10. The sample analyzer (10) of any one of claims 1 to 9, wherein
the processing unit (201) combines a plurality of the fluorescence images taken by the imaging unit (154), based on the one cell, and causes the display unit (203) to display an obtained composite image.

11. A sample analyzing method for analyzing a measurement specimen (21) containing a plurality of kinds of target substances each labeled with a fluorescent dye, the sample analyzing method comprising:
setting information on colors of fluorescence (λ21, ... λ25) which is a variable parameter such that the information on the colors of fluorescence (λ21, ... λ25) corresponds to the plurality of kinds of target substances;
flowing, through a flow cell (110), the measurement specimen (21);
applying light (λ11, ... λ16) to the measurement specimen (21) flowing through the flow cell (110) to generate, from the fluorescent dye, a plurality of kinds of fluorescences (λ21, ... λ25), having different wavelengths;
taking an image of the fluorescence (λ21, ... λ25) generated from a cell in the measurement specimen (21) flowing through the flow cell (110), for each color of the fluorescence (λ21, ... λ25), and generating a fluorescence image; and
analyzing the target substances based on the fluorescence image and the set information on the colors of fluorescences (λ21, ... λ25);
wherein the analyzing the target substances comprises:
receiving information on colors of fluorescences (λ21, ... λ25) corresponding to the respective target substances;
obtaining a positive pattern and a negative pattern based on the information on colors of fluorescences (λ21, ... λ25), the positive pattern and the negative pattern being bright point patterns;
extracting a fluorescence region from the fluorescence image and obtaining a bright point pattern of the cell based on the fluorescence region and the information on colors of fluorescences (λ21, ... λ25); and
determining whether the cell is positive or negative by comparing the bright point pattern of the cell with the positive pattern and the negative pattern.

## Patentansprüche

1. Probenanalysator (10) zum Analysieren eines Messprobestücks (21), das eine Vielzahl von Arten von Zielsubstanzen enthält, jede mit einem fluoreszierenden Farbstoff markiert, wobei der Probenanalysator (10) umfasst:
eine Messeinheit (100), umfassend:
eine Durchflusszelle (110), die dem Messprobestück (21) ermöglicht, dort hindurch zu fließen;
eine Lichtquelle (121, ... 126) , konfiguriert, um Licht (λ11, ... λ16) auf das Messprobestück (21) anzuwenden, das durch die Durchflusszelle (110) fließt, um aus dem fluoreszierenden Farbstoff eine Vielzahl von Arten von Fluoreszenzen (λ21, ... λ25) zu erzeugen, die unterschiedliche Wellenlängen aufweisen;
eine Abbildungseinheit (154), konfiguriert, um für jede Farbe der Fluoreszenz (λ21, ... λ25) ein Bild der Fluoreszenz (λ21, ... λ25) aufzunehmen, die von einer Zelle in dem Messprobestück (21) erzeugt wird, das durch die Durchflusszelle (110) fließt, und ein Fluoreszenzbild zu erzeugen; und
eine Verarbeitungseinheit (201), konfiguriert, um die Zielsubstanzen auf Grundlage des Fluoreszenzbilds und Informationen zu Farben von Fluoreszenzen (λ21, ... λ25) zu analysieren, die eingestellt ist, variabel zu sein, um der Vielzahl von Arten von Zielsubstanzen zu entsprechen,
wobei das Analysieren der Zielsubstanzen umfasst:
Empfangen von Informationen zu Farben von Fluoreszenzen (λ21, ... λ25) entsprechend den jeweiligen Zielsubstanzen;
Erhalten eines Positivmusters und eines Negativmusters auf Grundlage der Informationen zu Farben von Fluoreszenzen (λ21, ... λ25), wobei das Positivmuster und das Negativmuster Muster heller Punkte sind;
Extrahieren eines Fluoreszenzbereichs aus dem Fluoreszenzbild und Erhalten eines Musters heller Punkte der Zelle auf Grundlage des Fluoreszenzbereichs und der Informationen zu Farben von Fluoreszenzen (λ21, ... λ25); und
Bestimmen, ob die Zelle positiv oder negativ ist, durch Vergleichen des Musters heller Punkte der Zelle mit dem Positivmuster und dem Negativmuster.

2. Probenanalysator (10) nach Anspruch 1, umfassend eine Anzeigeeinheit (203), wobei
die Verarbeitungseinheit (201) die Anzeigeeinheit (203) veranlasst, einen Eingabebildschirm zum Empfangen von Eingabe der Informationen zu der Farbe der Fluoreszenz (λ21, ... λ25) derart anzuzeigen, dass die Informationen zu der Farbe der Fluoreszenz (λ21, ... λ25) der Zielsubstanz entspricht.

3. Probenanalysator (10) nach Anspruch 2, wobei der Eingabebildschirm eine Vielzahl von Sets umfasst, jeweils einschließend ein Element für die Zielsubstanz und ein Element zum Eingeben der Informationen zu der Farbe der Fluoreszenz (λ21, ... λ25).

4. Probenanalysator (10) nach Anspruch 2 oder 3, wobei
die Zielsubstanz mit dem Messelement in Beziehung steht,
die Verarbeitungseinheit (201) Eingabe von dem Messelement empfängt, und
der Eingabebildschirm ein Set eines Elements für die Zielsubstanz, das dem eingegebenen Messelement entspricht, und ein Element zum Eingeben der Informationen zu der Farbe der Fluoreszenz (λ21, ... λ25) umfasst.

5. Probenanalysator (10) nach einem der Ansprüche 1 bis 4, umfassend eine Informationserhaltungseinheit (205), konfiguriert, um Identifikationsinformationen zu erhalten, wobei
die Verarbeitungseinheit (201) Eingabe der Informationen zu der Farbe der Fluoreszenz (λ21, ... λ25) auf Grundlage der Identifikationsinformationen empfängt, die von der Informationserhaltungseinheit (205) erhalten werden.

6. Probenanalysator (10) nach Anspruch 5, umfassend eine Speichereinheit (202), konfiguriert, um die Informationen zu der Farbe der Fluoreszenz (λ21, ... λ25) derart zu speichern, dass die Informationen zu der Farbe der Fluoreszenz (λ21, ... λ25) den Identifikationsinformationen entsprechen, wobei
die Verarbeitungseinheit (201) Eingabe der Informationen zu der Farbe der Fluoreszenz (λ21, ... λ25), die der Zielsubstanz entsprechen, durch Lesen der Informationen zu der Farbe der Fluoreszenz (λ21, ... λ25) aus der Speichereinheit (202) empfängt, die den Identifikationsinformationen entsprechen, die von der Informationserhaltungseinheit (205) erhalten werden.

7. Probenanalysator (10) nach Anspruch 6, wobei
die Speichereinheit (202) die Vielzahl von Arten von Zielsubstanzen, die mit dem Messelement in Beziehung stehen, derart speichert, dass die Vielzahl von Arten von Zielsubstanzen den Identifikationsinformationen entsprechen, und
die Verarbeitungseinheit (201) Eingabe von Informationen zu den Farben der Fluoreszenzen (λ21, ... λ25), die der Vielzahl von Arten von Zielsubstanzen entsprechen, die mit dem Messelement in Beziehung stehen, durch Lesen, aus der Speichereinheit (202), des Messelements, das den Identifikationsinformationen entspricht, die von der Informationserhaltungseinheit (205) erhalten werden, und der Informationen zu den Farben der Fluoreszenzen (λ21, ... λ25), die den Identifikationsinformationen entsprechen, empfängt.

8. Probenanalysator (10) nach einem der Ansprüche 1 bis 7, wobei
die Informationen zu der Farbe der Fluoreszenz (λ21, ... λ25) mindestens eins eines Farbennamens der Fluoreszenz, einer Wellenlänge der Fluoreszenz (λ21, ... λ25), einer Art von Fluoreszenzkennzeichnung und einer Art eines Reagenzes auf Grundlage der Fluoreszenzkennzeichnung darstellen.

9. Probenanalysator (10) nach einem der Ansprüche 1 bis 8, umfassend eine Anzeigeeinheit (203), wobei
die Verarbeitungseinheit (201) die Anzeigeeinheit (203) veranlasst, das durch die Abbildungseinheit (154) aufgenommene Fluoreszenzbild anzuzeigen.

10. Probenanalysator (10) nach einem der Ansprüche 1 bis 9, wobei
die Verarbeitungseinheit (201) eine Vielzahl der von der Abbildungseinheit (154) aufgenommenen Fluoreszenzbilder, auf Grundlage der einen Zelle, kombiniert und die Anzeigeeinheit (203) veranlasst, ein erhaltenes Kompositbild anzuzeigen.

11. Probenanalysierverfahren zum Analysieren eines Messprobestücks (21), das eine Vielzahl von Arten von Zielsubstanzen enthält, jede mit einem fluoreszierenden Farbstoff gekennzeichnet, wobei das Probenanalysierverfahren umfasst:
Einstellen von Informationen zu Farben von Fluoreszenz (λ21, ... λ25), die variable Parameter sind, derart, dass die Informationen zu den Farben von Fluoreszenz (λ21, ... λ25) der Vielzahl von Arten von Zielsubstanzen entsprechen;
Fließenlassen, durch eine Durchflusszelle (110) hindurch, des Messprobestücks (21);
Anwenden von Licht (λ11, ... λ16) auf das Messprobestück (21), das durch die Durchflusszelle (110) hindurch fließt, um aus dem fluoreszierenden Farbstoff eine Vielzahl von Arten von Fluoreszenzen (λ21, ... λ25) zu erzeugen, die unterschiedliche Wellenlängen aufweisen;
Aufnehmen eines Bilds der Fluoreszenz (λ21, ... λ25), die von einer Zelle in dem Messprobestück (21), das durch die Durchflusszelle (110) hindurch fließt, erzeugt wird, für jede Farbe der Fluoreszenz (λ21, ... λ25), und Erzeugen eines Fluoreszenzbilds; und
Analysieren der Zielsubstanz auf Grundlage des Fluoreszenzbilds und der eingestellten Informationen zu den Farben von Fluoreszenzen (λ21, ... λ25);
wobei das Analysieren der Zielsubstanzen umfasst:
Empfangen von Informationen zu Farben von Fluoreszenzen (λ21, ... λ25) entsprechend den jeweiligen Zielsubstanzen;
Erhalten eines Positivmusters und eines Negativmusters auf Grundlage der Informationen zu Farben von Fluoreszenzen (λ21, ... λ25), wobei das Positivmuster und das Negativmuster Muster heller Punkte sind;
Extrahieren eines Fluoreszenzbereichs aus dem Fluoreszenzbild und Erhalten eines Musters heller Punkte der Zelle auf Grundlage des Fluoreszenzbereichs und der Informationen zu Farben von Fluoreszenzen (λ21, ... λ25); und
Bestimmen, ob die Zelle positiv oder negativ ist, durch Vergleichen des Musters heller Punkte der Zelle mit dem Positivmuster und dem Negativmuster.

## Revendications

1. Analyseur d'échantillon (10) pour analyser un spécimen de mesure (21) contenant une pluralité de types de substances cibles, chacune marquée avec un colorant fluorescent, l'analyseur d'échantillon (10) comprenant :
une unité de mesure (100) comprenant :
une cellule d'écoulement (110) permettant au spécimen de mesure (21) de s'écouler à travers celle-ci ;
une source de lumière (121, 126) configurée pour appliquer une lumière (λ11, ... λ16) sur le spécimen de mesure (21) s'écoulant à travers la cellule d'écoulement (110) pour générer, à partir du colorant fluorescent, une pluralité de types de fluorescences (λ21, ... λ25) présentant différentes longueurs d'onde ;
une unité d'imagerie (154) configurée pour prendre une image de la fluorescence (λ21, ... λ25) générée à partir d'une cellule dans le spécimen de mesure (21) s'écoulant à travers la cellule d'écoulement (110), pour chaque couleur de la fluorescence (λ21, ... λ25), et générer une image de fluorescence ; et
une unité de traitement (201) configurée pour analyser les substances cibles sur la base de l'image de fluorescence et d'informations sur les couleurs de fluorescences (λ21, ... λ25), qui sont établies de manière à être variables afin de correspondre à la pluralité de types de substances cibles,
dans lequel l'analyse des substances cibles comprend :
la réception d'informations sur les couleurs de fluorescences (λ21, ... λ25) correspondant aux substances cibles respectives ;
l'obtention d'un motif positif et d'un motif négatif sur la base des informations sur les couleurs de fluorescences (λ21, ... λ25), le motif positif et le motif négatif étant des motifs de points lumineux ;
l'extraction d'une région de fluorescence à partir de l'image de fluorescence et l'obtention d'un motif de points lumineux de la cellule sur la base de la région de fluorescence et des informations sur les couleurs de fluorescences (λ21, ... λ25) ; et
la détermination si la cellule est positive ou négative en comparant le motif de points lumineux de la cellule avec le motif positif et le motif négatif.

2. Analyseur d'échantillon (10) selon la revendication 1, comprenant une unité d'affichage (203), dans lequel
l'unité de traitement (201) amène l'unité d'affichage (203) à afficher un écran d'entrée pour recevoir une entrée des informations sur la couleur de la fluorescence (λ21, ... λ25) de sorte que les informations sur la couleur de la fluorescence (λ21, ... λ25) correspondent à la substance cible.

3. Analyseur d'échantillon (10) selon la revendication 2, dans lequel l'écran d'entrée comprend une pluralité d'ensembles incluant chacun un élément pour la substance cible et un élément pour enter les informations sur la couleur de la fluorescence (λ21, ... λ25).

4. Analyseur d'échantillon (10) selon la revendication 2 ou 3, dans lequel
la substance cible est en rapport avec l'élément de mesure,
l'unité de traitement (201) reçoit une entrée de l'élément de mesure, et
l'écran d'entrée comprend un ensemble d'un élément pour la substance cible correspondant à l'élément de mesure entré, et d'un élément pour entrer les informations sur la couleur de la fluorescence (λ21, ... λ25).

5. Analyseur d'échantillon (10) selon l'une quelconque des revendications 1 à 4, comprenant une unité d'obtention d'informations (205) configurée pour obtenir des informations d'identification, dans lequel
l'unité de traitement (201) reçoit une entrée des informations sur la couleur de la fluorescence (λ21, ... λ25), sur la base des informations d'identification obtenues par l'unité d'obtention d'informations (205).

6. Analyseur d'échantillon (10) selon la revendication 5, comprenant une unité de stockage (202) configurée pour stocker les informations sur la couleur de la fluorescence (λ21, ... λ25) de sorte que les informations sur la couleur de la fluorescence (λ21, ... λ25) correspondent aux informations d'identification, dans lequel
l'unité de traitement (201) reçoit une entrée des informations sur la couleur de la fluorescence (λ21, ... λ25) qui correspondent à la substance cible en lisant, à partir de l'unité de stockage (202), les informations, sur la couleur de la fluorescence (λ21, ... λ25), qui correspondent aux informations d'identification obtenues par l'unité d'obtention d'informations (205).

7. Analyseur d'échantillon (10) selon la revendication 6, dans lequel
l'unité de stockage (202) stocke la pluralité de types de substances cibles en rapport avec l'élément de mesure de sorte que la pluralité de types de substances cibles correspondent aux informations d'identification, et
l'unité de traitement (201) reçoit une entrée des informations sur les couleurs des fluorescences (λ21, ... λ25) qui correspondent à la pluralité de types de substances cibles en rapport avec l'élément de mesure, en lisant, à partir de l'unité de stockage (202), l'élément de mesure qui correspond aux informations d'identification obtenues par l'unité d'obtention d'informations (205), et les informations, sur les couleurs des fluorescences (λ21, ... λ25), correspondant aux informations d'identification.

8. Analyseur d'échantillon (10) selon l'une quelconque des revendications 1 à 7, dans lequel
les informations sur la couleur de la fluorescence (λ21, ... λ25) représentent au moins l'un parmi un nom de couleur de la fluorescence, une longueur d'onde de la fluorescence (λ21, ... λ25), un type de marquage par fluorescence, et un type de réactif basé sur le marquage par fluorescence.

9. Analyseur d'échantillon (10) selon l'une quelconque des revendications 1 à 8, comprenant une unité d'affichage (203), dans lequel
l'unité de traitement (201) amène l'unité d'affichage (203) à afficher l'image de fluorescence prise par l'unité d'imagerie (154).

10. Analyseur d'échantillon (10) selon l'une quelconque des revendications 1 à 9, dans lequel
l'unité de traitement (201) combine une pluralité des images de fluorescence prises par l'unité d'imagerie (154), sur la base d'une cellule, et amène l'unité d'affichage (203) à afficher une image composite obtenue.

11. Procédé d'analyse d'échantillon pour analyser un spécimen de mesure (21) contenant une pluralité de types de substances cibles, chacune marquée avec un colorant fluorescent, le procédé d'analyse d'échantillon comprenant :
l'établissement d'informations sur des couleurs de fluorescence (λ21, ... λ25) qui sont un paramètre variable de sorte que les informations sur les couleurs de fluorescence (λ21, ... λ25) correspondent à la pluralité de types de substances cibles ;
l'écoulement, à travers une cellule d'écoulement (110), du spécimen de mesure (21) ;
l'application d'une lumière (λ11, ... λ16) sur le spécimen de mesure (21) s'écoulant à travers la cellule d'écoulement (110) pour générer, à partir du colorant fluorescent, une pluralité de types de fluorescences (λ21, ... λ25) présentant différentes longueurs d'onde ;
la prise d'une image de la fluorescence (λ21, ... λ25) générée à partir d'une cellule dans le spécimen de mesure (21) s'écoulant à travers la cellule d'écoulement (110), pour chaque couleur de la fluorescence (λ21, ... λ25), et la génération d'une image de fluorescence ; et
l'analyse des substances cibles sur la base de l'image de fluorescence et des informations établies sur les couleurs de fluorescences (λ21, ... λ25) ;
dans lequel l'analyse des substances cibles comprend :
la réception d'informations sur les couleurs de fluorescences (λ21, ... λ25) correspondant aux substances cibles respectives ;
l'obtention d'un motif positif et d'un motif négatif sur la base des informations sur les couleurs de fluorescences (λ21, ... λ25), le motif positif et le motif négatif étant des motifs de points lumineux ;
l'extraction d'une région de fluorescence à partir de l'image de fluorescence et l'obtention d'un motif de points lumineux de la cellule sur la base de la région de fluorescence et des informations sur les couleurs de fluorescences (λ21, ... λ25) ; et
la détermination si la cellule est positive ou négative en comparant le motif de points lumineux de la cellule avec le motif positif et le motif négatif.
